# EUROPEAN PATENT APPLICATION

(11) **EP 2 336 769 A1**
(43) Date of publication of application: **22.06.2011**
(21) Application number: 09179778.7
(22) Date of filing: 18.12.2009
(51) Int. Cl.: G01N 33/50, G01N 33/564

(54) **Trigger assay for differentiating between rheumatic and non-rheumatic disorders**

(71) Applicant: F. Hoffmann-La Roche AG, 4070 Basel (CH); Roche Diagnostics GmbH, 68305 Mannheim (DE)
(72) Inventor: Karl, Johann, 82380 Peissenberg (DE); Klause, Ursula, 82380 Peissenberg (DE); Grunert, Veit-Peter, 81479 München (DE)
(74) Representative: Schiweck, Weinzierl & Koch

(57) **Abstract**

The present invention provides for a method for excluding a non-rheumatic disorder as cause of a musculoskeletal complaint of a subject comprising determining as to whether at least one autoantibody or antigen which is indicative for at least one rheumatic disorder is present in a sample obtained from the subject, wherein the presence of said autoantibody or antigen allows to exclude a non-rheumatic disorder as cause of a musculoskeletal complaint of a subject. Accordingly, it provides for a method that triggers the next steps in diagnosing the cause of a musculoskeletal complaint of a subject - either further diagnosis of a rheumatic disorder or of a non-rheumatic disorder to confirm the initial diagnosis. Also provided is a kit for excluding a non-rheumatic disorder as cause of a musculoskeletal complaint of a subject as well as uses of the kit.

## Description

The present invention provides for a method for excluding a non-rheumatic disorder as cause of a musculoskeletal complaint of a subject comprising determining as to whether at least one autoantibody or antigen which is indicative for at least one rheumatic disorder is present in a sample obtained from the subject, wherein the presence of said autoantibody or antigen allows to exclude a non-rheumatic disorder as cause of a musculoskeletal complaint of a subject. Accordingly, it provides for a method that triggers the next steps in diagnosing the cause of a musculoskeletal complaint of a subject - either further diagnosis of a rheumatic disorder or of a non-rheumatic disorder to confirm the initial diagnosis. Also provided is a kit for excluding a non-rheumatic disorder as cause of a musculoskeletal complaint of a subject as well as uses of the kit.

Musculoskeletal complaints cause each year many patients to seek medical care, initially prompting a thorough clinical history and physical examination. It is estimated that musculoskeletal complaints account worldwide for millions of outpatient visits at doctors per year. Musculoskeletal disorders are among the most frequent causes of physical disability. People with musculoskeletal pain sometimes complain that their entire bodies ache. Their muscles might feel like they have been pulled or overworked. Sometimes the muscles twitch or burn. Symptoms vary from person to person, but the common symptoms are pain, fatigue and sleep disturbances.

Musculoskeletal disorders are common diseases affecting a large human population. They may be caused by rheumatic disorders. However, they may also be caused by non-rheumatic disorders. They cause pain of various types such as neck pain, limb pain, low back pain, joint pain, chronic widespread pain and are a major reason for consultation in primary care. Musculoskeletal pain can be caused by damage to bones, joints, muscles, tendons, ligaments, bursae, or nerves. Injuries are the most common cause. If no injury has occurred or if pain persists for more than a few days then another cause is often responsible, for example, osteoarthritis or an autoimmune disease.

The increasing prevalence of musculoskeletal pain has been described as an epidemic. Pain is the chief symptom of most musculoskeletal disorders and it is frequently caused by injury or inflammation. Besides pain, other symptoms such as stiffness, tenderness, weakness and swelling or deformity of affected parts are manifestations of musculoskeletal disorders.

Virtually all joint injuries and diseases produce a stiff, aching pain, often referred to as "arthritic" pain. The pain is worse when the joint is moved and may range from mild to severe. With some conditions, there may be swelling of the joint along with the pain. Joint inflammation (arthritis) is a common cause of joint pain. There are many types of arthritis, including rheumatoid and other types of inflammatory arthritis, osteoarthritis, infectious arthritis, and arthritis due to gout or pseudogout. Other causes of joint pain include autoimmune and vasculitic disorders (for example, systemic lupus erythematosus, polymyalgia rheumatica, and polyarteritis nodosa), avascular necrosis of bone, and injuries (for example, dislocations, sprains, and fractures affecting the portion of the bone inside the joint). Sometimes, pain originating in structures near the joint, such as tendons and bursae, seems to be coming from the joint.

Some musculoskeletal disorders cause pain by compressing nerves. These conditions include the "tunnel syndromes" (for example, carpal tunnel syndrome, cubital tunnel syndrome, and tarsal tunnel syndrome). The pain tends to radiate along the path supplied by the nerve and may be burning.

Bursal pain can be caused by bursitis or fibromyalgia. Usually, bursal pain is worse with movement involving the bursa. There may be swelling.

As mentioned above, the causes of musculoskeletal pain are varied. Muscle tissue can be damaged with the wear and tear of daily activities. Trauma to an area (such as jerking movements, auto accidents, falls, fractures, sprains, dislocations, and direct blows to the muscle) also can cause musculoskeletal pain. Other causes of pain include postural strain, repetitive movements, overuse, and prolonged immobilization. Changes in posture or poor body mechanics might bring about spinal alignment problems and muscle shortening, therefore causing other muscles to be misused and become painful. However, musculoskeletal pain can also be caused by autoimmune disorders such as rheumatoid arthritis, sj6gren's syndrome (SS), systemic lupus erythematosus (SLE), systemic scleroderma, CREST syndrome, undifferentiated connective tissue disease (UCTD), polymyositis (PM), dermatomyositis (DM) or antineutrophil cytoplasmic autoantibodies (ANCA)-associated vasculitis can be named as autoimmune-mediated musculoskeletal disorders. These are merely some examples of autoimmune disorders that may cause musculoskeletal disorders and there are many more.

Autoimmune disorders are diseases caused by the body producing an inappropriate immune response against its own tissues. Sometimes the immune system will cease to recognize one or more of the body's normal constituents as "self" and will create autoantibodies - antibodies that attack its own cells, tissues, and/or organs. This causes inflammation and damage and it leads to autoimmune disorders. In autoimmune disorders, both humoral and cellular immunity plays a pathophysiological role in the development of the disease. Damage to tissue and organs appears to result from the presence and infiltration of autoantibodies and/or auto-reactive T cells.

The cause of autoimmune diseases is unknown, but it appears that there is an inherited predisposition to develop autoimmune disease in many cases. In a few types of autoimmune disease (such as rheumatic fever), a bacteria or virus triggers an immune response, and the antibodies or T-cells attack normal cells because they have some part of their structure that resembles a part of the structure of the infecting microorganism. Autoimmune disorders fall into two general types: those that damage many organs (systemic autoimmune diseases) and those where only a single organ or tissue is directly damaged by the autoimmune process (localized). However, the distinctions become blurred as the effect of localized autoimmune disorders frequently extends beyond the targeted tissues, indirectly affecting other body organs and systems.

In autoimmune-mediated musculoskeletal disorders the exact "target" of autoantibodies and autoreactive T cells is not known or, at best, it is unclear. However, it is known that autoimmune disorders such as RA, SLE or SS cause musculoskeletal complaints.

In case of autoimmune-mediated musculoskeletal disorders a wide array of antibody specificities has been described. Determination of autoantibodies provides crucial information to establish the diagnosis of the disorder. In addition, the determination of these antibodies may have prognostic value or may be used to monitor response to treatment or to predict relapse of disease. The clinically most relevant assays include rheumatoid factor (RF), anti-cyclic citrullinated antibody (anti-CCP), antinuclear autoantibodies (ANA), anti-double-stranded DNA antibodies, antibodies to extractable nuclear antigens (ENA), and antineutrophil cytoplasmic autoantibodies (ANCA). However, when the doctor uses the available autoantibody tests, he has to be aware of their fallacies. It is of great importance to perform these tests in the context of a valid clinical suspicion and not merely as screening tests. In addition, the doctor has to be aware that repeated measurements of autoantibodies in specific musculoskeletal disorders are useful only if the relevance of these autoantibodies for disease monitoring has been demonstrated.

Accordingly, specific assays for detecting autoantibodies indicative for autoimmune disorders causing symptoms of rheumatoid disorders such as RA or SLE can been applied. These assays make use of the detection of autoantibodies against predetermined autoantigens such as CCP, ANA or ANCA (see, for example, WO 2007/039280 or Feng et al. (2004), Clin. Chem. 50: 416-422). Therefore, the so far applied assays serve for the purpose of specifically diagnosing an autoimmune disorder, whereby the doctor has already an intuition or suspicion based on physical examination, imaging tests and the patient's medical history.

Accordingly, so far applied assays for testing autoimmune-mediated musculoskeletal disorders merely provide for the differential diagnosis and can thus be used by physicians or other clinicians to diagnose the specific disease in a patient. Yet, so far, no one fits all assay is available.

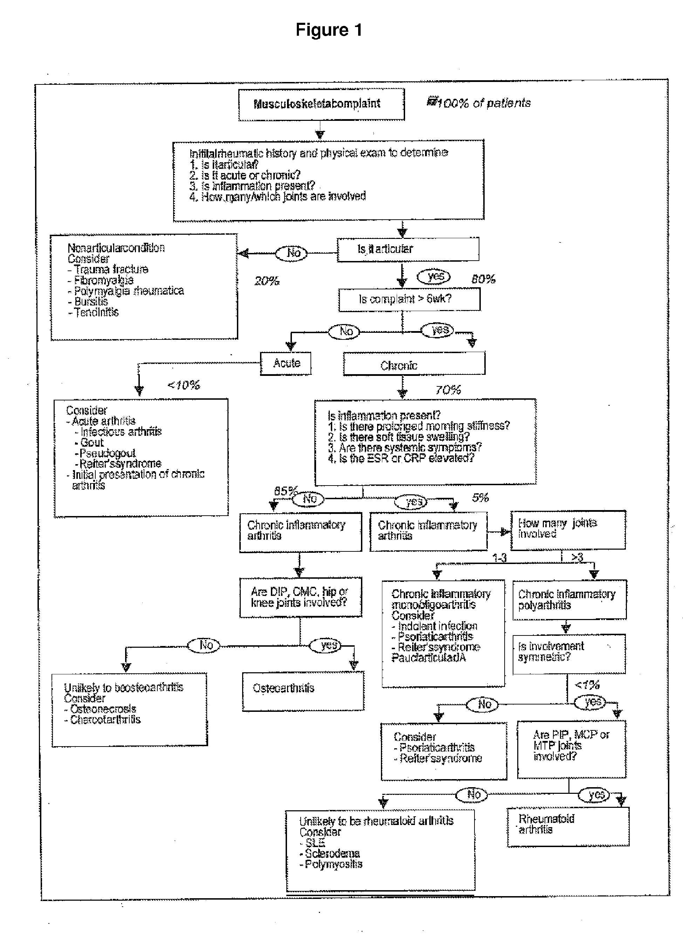

In sum, due to their rather complex etiology, diagnosing the cause of a musculoskeletal complaint is difficult. Proper physical examination, various tests, imaging, nerve tests, autoantibody tests, etc. are to be conducted before a specific cause for the musculoskeletal complaint is determined. Since the diagnosis is very complex and many causes could be responsible for a musculoskeletal complaint, the diagnosis requires a classification, whereby at each stage new bifurcations occur which is apparent from Figure 1. For a general practitioner who is usually the first contact point for a patient it is almost impossible to make an ad hoc reliable diagnosis. In fact, for a general practitioner it is thus quite difficult to differentiate, for example, between various arthritic disorders since patients may have a certain amount of overlap both with regard to clinical symptoms. Indeed, SLE, RA and SS cause similar symptoms. Moreover, osteoarthritis is also an arthritic disorder, though not being caused by an autoimmune musculoskeletal disorder. This is just an example to illustrate the complexity of the diagnosis of a musculoskeletal complaint.

The above being said, the most important step in diagnosing the cause of a musculoskeletal complaint is the initial step in the hierarchy of the classification, i.e., the determination whether the cause of a musculoskeletal complaint is a non-autoimmune disorder or an autoimmune-mediated musculoskeletal disorder.

Thus, it is very important to distinguish at the earliest possible stage of a patient's medical history between a non-rheumatic disorder and a rheumatic disorder, for example, caused by an autoimmune-mediated musculoskeletal disorder. Otherwise the patient may be subject to false treatment which may even worsen the situation. For example, it may happen that patients are initially diagnosed by the general practitioner as having a non-autoimmune musculoskeletal disorder which causes musculoskeletal complaints, while in reality these patients have an autoimmune-mediated musculoskeletal disorder. Likewise, patients initially diagnosed as having an autoimmune-mediated musculoskeletal disorder may in reality have a non-autoimmune musculoskeletal disorder.

Hence, given the above, it would be desirable to have available an assay which allows a general practitioner to discriminate/separate a non-rheumatic disorder from a rheumatic disorder as the cause of musculoskeletal complaints or vice versa, i.e. to discriminate a rheumatic disorder from a non-rheumatic disorder as the cause of musculoskeletal complaints at the top of a classification hierarchy in order to transfer the patient to the right specialist (i.e., rheumatologist) and to subject a patient to the most appropriate treatment.. Such an assay "triggers" off the final diagnosis and the treatment of a patient by an orthopedic surgeon or a rheumatologist.

Accordingly, the technical problem of the present invention is to comply with the need described above.

The present invention addresses this need and thus provides as a solution to the technical problem methods, uses and means for excluding a non-rheumatic disorder as cause of a musculoskeletal complaint or for excluding a rheumatic disorder as cause of a musculoskeletal complaint, respectively. These embodiments are characterized and described herein, shown in the Figures, illustrated in the Examples, and reflected in the claims.

The present inventors have recognized that it is of utmost importance to provide a doctor, for example, a general practitioner at the earliest possible point in time, i.e., when a subject consults a doctor because of a musculoskeletal complaint, with means and methods which allow excluding a non-rheumatic disorder as cause of a musculoskeletal complaint or, vice versa, with methods which allow excluding an rheumatic disorder as cause of a musculoskeletal complaint, respectively.

Otherwise, it may inevitably occur that the subject is not treated most appropriately that, however, may even worsen the subject's musculoskeletal complaint. For example, if a subject suffering from a musculoskeletal complaint caused by rheumatoid arthritis is not treated as soon as possible with appropriate means because of a false-negative diagnosis, the rheumatoid arthritis may become worse and causes even more irreversible damage and thus a health economical problem.

Up to now, for determining as to whether a rheumatic disorder may be the cause of a musculoskeletal complaint, only differential diagnostic assays have been applied. These assays start from the presumption that a subject suffers from such a rheumatic disorder, even more, it is presumed, on the basis of the subject's medical history, which rheumatic disorder the subject may have. In other words, so far diagnosis was made for the purpose of confirming an assumed rheumatic disorder. Thus, the so far applied assays contain specific analytes or marker panels of autoantigens which are indicative for a specific rheumatic disorder such as rheumatoid arthritis.

However, in spite of the availability of differential diagnostic assays, a doctor additionally requires clinical results in order to improve or verify the differential diagnosis. For a general practitioner it is in most cases nearly impossible to readily distinguish between a rheumatic disorder and a non-rheumatic disorder as cause of a musculoskeletal complaint. However, from a health economics point of view it would be of high interest to make this distinction as early as possible so that a subject having a musculoskeletal complaint is committed to the appropriate doctor - either a rheumatologist which is also specialized in rheumatic disorders (rheumatologist) or an orthopedic surgeon.

Indeed, functionally relevant damage caused by chronic systemic rheumatic disorders can be reduced or sometimes avoided by early initiation of the appropriate treatment. This, however, requires a correct diagnosis which makes treatment as early as possible. Due to the often uncharacteristic symptoms at the onset of this kind of disorders, early diagnosis represents a diagnostic challenge.

The present inventors have recognized that, in spite of the availability of many differential diagnostic assays and methods for diagnosing a specific rheumatic disorder which causes musculoskeletal complaints and to the best of their knowledge, no assay was available which allows a doctor to immediately discriminate between a rheumatic disorder and a non-rheumatic disorder as cause of a musculoskeletal complaint, i.e., the present inventors provide, so to say, a doctor's office discrimination device. In fact, due to the often uncharacteristic symptoms at the onset of rheumatic disorder such as autoimmune-mediated musculoskeletal disorders, they are very often prematurely disregarded and non-rheumatic disorder are assumed as the cause of musculoskeletal complaints.

Thus, there is a need, in particular for general practitioners as well as for medical specialists to have available means and methods which can be applied as the initial step ("first aid") in finding out as to whether or not a non-rheumatic disorder or, in the reverse, whether or not a rheumatic disorder can be excluded as cause for a musculoskeletal complaint.

Accordingly, in a first aspect the present invention relates to a method for excluding a non-rheumatic disorder as cause of a musculoskeletal complaint of a subject comprising determining as to whether at least one autoantibody or antigen which is indicative for at least one rheumatic disorder is present in a sample obtained from the subject, wherein the presence of said autoantibody or antigen allows to exclude a non-rheumatic disorder as cause of a musculoskeletal complaint of a subject.

In an alterative and/or additional aspect the present invention relates to a method for excluding a rheumatic disorder as cause of a musculoskeletal complaint of a subject comprising determining as to whether at least one autoantibody or antigen which is indicative for at least one rheumatic disorder is absent in a sample obtained from the subject,
wherein the absence of said autoantibody or antigen allows to exclude an rheumatic disorder as cause of a musculoskeletal complaint of a subject.

Accordingly, the method of excluding of the present invention may also be seen as a method of elimination of or a method of discriminating or a method of separating between a non-rheumatic disorder and a rheumatic disorder, respectively, as cause of a musculoskeletal complaint, in particular at the onset (preferably early onset) of the musculoskeletal complaint.

Moreover, the method of the present invention is for triggering the further steps in diagnosing the cause of a musculoskeletal complaint of a subject - either further diagnosis of a rheumatic disorder or of a non-rheumatic disorder to confirm the initial diagnosis.

In fact, it is an advantage which is preferred that the method allows determining at the onset of a musculoskeletal complaint as to whether or not a non-rheumatic disorder can be excluded as cause of a musculoskeletal complaint of a subject.

Alternatively and/or additionally, the method allows determining at the onset, i.e. in the early phase of a musculoskeletal complaint as to whether or not a rheumatic disorder (or non-rheumatic disorder) can be excluded as cause of a musculoskeletal complaint of a subject.

"Excluding" in all its grammatical forms when used in the context of the methods, uses and kits of the present invention means that a non-rheumatic disorder or a rheumatic disorder, respectively, can be eliminated as cause of a musculoskeletal complaint of a subject or can be separated/discriminated from a non-rheumatic disorder or a rheumatic disorder, respectively. Of course, false-positive or false-negative results cannot be excluded since such results are, as is commonly known, an inherent property of a diagnostic method. However, as is described herein the threshold values which are decisive about a positive or negative result are determined as careful as possible. Accordingly, in case of doubt at the practitioner's end, the diagnostic test of the present invention can be repeated and/or may be supplemented by additional tests known in the art.

A "subject" when used herein includes mammalian and non-mammalian subjects. "Mammal" for purposes of treatment refers to any animal classified as a mammal, including human, domestic and farm animals, nonhuman primates, and any other animal that has mammary tissue. A mammal includes human, rodents such as mouse, rat or rabbit, dog, cat, chimpanzee, horse, pig, etc., with human being preferred. A subject also includes human and veterinary patients, with human patients being preferred.

A "patient" is an individual subject, preferably having a musculoskeletal complaint which is tested for the presence or absence of a rheumatic disorder or a non-rheumatic disorder, respectively. A patient tested for a rheumatic disorder or a non-rheumatic disorder can have one or more indicators of such a disorder as described herein, or can be screened for the presence or absence of said disorder in the absence of any indicators of said disorder. As used herein an "individual suspected of having said disorder" or "subject suspected of having said disorder" can have one or more indicators of said disorder or is at an increased risk of developing said disorder due to age, environmental and/or nutritional factors, or genetic factors or can be part of a population routinely screened for said disorder in the absence of any indicators of said disorder.

In accordance with the present invention by the term "sample" is intended any biological sample obtained from a subject, cell line, tissue culture, or other source containing autoantibodies, polynucleotides or polypeptides or portions thereof. Biological samples include body fluids (such as blood, serum, plasma, urine, saliva, synovial fluid and spinal fluid) and tissue sources found to contain autoantibodies. Methods for obtaining tissue biopsies and body fluids from subjects are well known in the art. Generally, a biological sample which includes proteins, in particular antigens, autoantibodies and polynucleotides is preferred as a source.

Other preferred samples are whole blood, serum, plasma or synovial fluid, with plasma or serum being most preferred.

"Presence" (in all its grammatical forms) of an autoantibody or antigen means that an autoantibody or antigen which is indicative for at least one rheumatic disorder is immunologically detectable. Accordingly, the autoantibody is bound to an autoantigen contained in a test area as described herein. Likewise, the antigen is bound by an antibody contained in a test area as described herein.

Any immunoassay known in the art can be used for immunologically detecting the binding of an autoantibody to an autoantigen or the binding of an antigen to an antibody. Immunoassays are well known to the skilled artisan. Methods for carrying out such assays as well as practical applications and procedures are summarized in related textbooks. Examples of related textbooks are Tijssen, P., In: Practice and theory of enzyme immunoassays, Burdon, R.H. and v. Knippenberg, P. H. (eds.), Elsevier, Amsterdam (1990), pp. 221-278, and various volumes of Colowick, S. P. and Caplan, N.O. (eds.), Methods in Enzymology, Academic Press, dealing with immunological detection methods, especially volumes 70, 73, 74, 84, 92 and 121.

Immunoassays, for example, include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, solid phase immune tests, and mass spectrometry such as SELDI-TOF, MALDI-TOF, or capillary electrophoresis-mass spectrometry (CE-MS).

Furthermore, suitable immunoassays include microplate ELISA-based methods, fully-automated or robotic immunoassays (available for example on EleCSyS™ or CobaS™ analyzers), and latex agglutination assays (available for example on Roche-Hitachi™ analyzers).

Typically, in the present invention an immunoassay is an assay in which an autoantibody specifically binds an autoantigen to provide for the detection and/or quantification of an autoantibody or an assay in which an antigen binds to an antibody to provide for the detection and/or quantification of said antigen.

"Immunologically detectable" when used herein preferably includes that a signal generated by the binding of an autoantibody to an autoantigen or by the binding of an antigen to an antibody as described herein is above a predetermined test-area specific threshold value, i.e., the signal generated by the binding of an autoantibody to an autoantigen or the binding of an antigen to an antibody is positive.

"Absence" (in all its grammatical forms) of an autoantibody means that an autoantibody or antigen which is indicative for at least one rheumatic disorder is not immunologically detectable. Accordingly, the autoantibody is not bound to an autoantigen contained in the test area as described herein. Likewise, "absence" means that the antigen is not bound by an antibody contained in the test area as described herein. As mentioned above, any immunoassay known in the art can be used.

"Is not immunologically detectable" when used herein preferably includes that a signal generated by the binding of an autoantibody to an autoantigen or the binding of an antigen to an antibody as described herein is below a predetermined test-area specific threshold value, i.e., the signal generated by the binding of an autoantibody to an autoantigen or by the binding of an antigen to an antibody is negative.

Accordingly, in a preferred aspect of the methods of the present invention a signal is classified as positive when it is above a predetermined test-area-specific cut-off value and is classified as negative when it is below a predetermined test-area-specific cut-off value, wherein a positive signal excludes a non-rheumatic disorder as cause of a musculoskeletal complaint.

Likewise, a negative signal excludes a rheumatic disorder as cause of a musculoskeletal complaint.

A "cut-off value" is the boundary value (or limit value) of the amount and/or concentration of an autoantibody or antigen that determines as to when a signal is positive or negative. In the present case, the cut-off value indirectly reflects the amount and/or concentration of an autoantibody or antigen, since on the test area a certain amount and/or concentration of an autoantigen or antibody is present to which autoantibodies or antigens contained in a sample from a subject bind.

Thus, a "signal" reflects the result of an autoantibody-autoantigen reaction which includes the binding of an autoantibody to an autoantigen present on the test area. Accordingly, the term "signal" includes the result of an autoantibody-autoantigen reaction which includes the binding of an autoantibody to an autoantigen present on the test area. Likewise, this term includes the result of an antigen-antibody reaction which includes the binding of an antigen to an antibody present on the test area.

If the cut-off value is exceeded (or above a predetermined threshold value), this is indicative of the presence of an autoantibody or antigen indicative for a rheumatic disorder and, thus, a signal or the result of an autoantibody-autoantigen reaction or antigen-antibody reaction is classified as positive.

However, if the cut-off value is deceeded (or below a predetermined threshold value), this is indicative of the absence of an autoantibody or antigen indicative for a rheumatic disorder and, thus, a signal or the result of an autoantibody-autoantigen reaction or antigen-antibody reaction is classified as negative.

A signal can be determined by way of the signal provided by the signal generating group of an autoantibody-specific receptor which is described herein below. The signal can be any signal which is detectable by, for example, spectroscopic, photochemical, biochemical, immunochemical, or chemical means as described herein below.

A signal can also be determined by way of the signal provided by the signal generating group of an antigen-specific receptor which is described herein below.

By using predetermined test-area specific threshold values it is possible to optimize these cut-off values according to the assay requirements. Therefore, the skilled artisan can shift the cut-off values to achieve a higher specificity or a higher sensitivity of the methods of the present invention and kits applying these methods. The threshold value or cut off value can be determined by different procedures as known from virological testing. The preferred embodiment is to measure a sufficient set of patient samples with rheumatic disorders ("diseased collective" or "disease group") and patient samples with non-rheumatic disorders ("control collective" or "control group" or "control sample") and to fix the cut-off as required for the clinical situation.

Preferably, cut off values are individually determined for each autoantigen or antibody that is preferably present on a test area. This means that for different test areas different cut off values are determined.

Accordingly, it is a preferred aspect of the methods of the present invention that said cut-off value is determined individually for each test area.

For example, a reference value may be determined as the median or as specific percentile (i.e., 0.90 or 0.95) of the measured levels in a population of individuals not suffering from a rheumatic disorder. Evaluating the levels in further individuals or patients, e.g. in cohort studies, can help to refine the known levels or ratios. Analogously, it is also possible to define and/or refine reference levels indicative of the presence of autoantibodies indicative for a rheumatic disorder.

Also, a level known in the art for a particular autoantibody may be used as a "reference value". The person skilled in the art is familiar with the concept of reference values (or "normal values") for biomarkers such as autoantibodies indicative for disorder a rheumatic disorder. In particular, the term reference value may relate to the actual value of the level in one or more control samples or it may relate to a value derived from the actual level in one or more control samples. Preferably, samples of at least 50, more preferably at least 100, more preferably at least 500, most preferably at least 1000 subjects.

In the simplest case, the reference value is the same as the level measured in the control sample or the average of the levels measured in a multitude of control samples. However, the reference value may also be calculated from more than one control sample. E.g., the reference value may be the arithmetic average of the level in control samples representing the control status (e.g. healthy, particular condition, or particular disease state). Preferably, the reference value relates to a range of values that can be found in a plurality of comparable control samples (control samples representing the same or similar disease status), e.g. the average one or more times the standard deviation.

Similarly, the reference value may also be calculated by other statistical parameters or methods, for example as a defined percentile of the level found in a plurality of control samples, e.g. a 90 %, 95 %, 97.5%, or 99 % percentile. The choice of a particular reference value may be determined according to the desired sensitivity, specificity or statistical significance (in general, the higher the sensitivity, the lower the specificity and vice versa). Calculation may be carried out according to statistical methods known and deemed appropriate by the person skilled in the art.

The terms "control" or "control sample" or the like are known to the person skilled in the art. Preferably, the "control" relates to an experiment or test carried out to provide a standard, against which experimental results (e.g. the measured level(s) in a subject) can be evaluated. In the present context, the standard preferably relates to the level of the autoantibody of interest indicative for a rheumatic disorder. Thus, a "control" is preferably a sample taken to provide such a standard. E.g., the control sample may be derived from one or more healthy subjects, or from one or more subjects representative of a non-rheumatic disorder.

It is a preferred embodiment of the invention to use an optimized multivariate cut-off for the underlying autoantibodies the presence or absence of which is to be determined in accordance with the methods of the present invention so as to discriminate state A from state B, e.g. the cause of the musculoskeletal complaint is caused by a rheumatic disorder or by a non-rheumatic disorder. Accuracy of a diagnostic method is best described by its receiver-operating characteristics (ROC) (see especially Zweig, M. H., and Campbell, G., Clin. Chem. 39 (1993) 561-577). The ROC graph is a plot of all of the sensitivity/specificity pairs resulting from continuously varying the decision thresh-hold over the entire range of data observed.

The clinical performance of a laboratory test depends on its diagnostic accuracy, or the ability to correctly classify subjects into clinically relevant subgroups. Diagnostic accuracy measures the test's ability to correctly distinguish two different conditions of the subjects investigated. Such conditions are for example health and disease or benign versus malignant disease.

In each case, the ROC plot depicts the overlap between the two distributions by plotting the sensitivity versus 1 - specificity for the complete range of decision thresholds. On the y-axis is sensitivity, or the true-positive fraction [defined as (number of true-positive test results)/(number of true-positive + number of false- negative test results)]. This has also been referred to as positivity in the presence of a disease or condition. It is calculated solely from the affected subgroup. On the x-axis is the false-positive fraction, or 1 - specificity [defined as (number of false- positive results)/(number of true-negative + number of false-positive results)]. It is an index of specificity and is calculated entirely from the unaffected subgroup. Because the true- and false-positive fractions are calculated entirely separately, by using the test results from two different subgroups, the ROC plot is independent of the prevalence of disease in the sample. Each point on the ROC plot represents a sensitivity/1-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test.

One convenient goal to quantify the diagnostic accuracy of a laboratory test is to express its performance by a single number. The most common global measure is the area under the ROC plot. By convention, this area is always > 0.5 (if it is not, one can reverse the decision rule to make it so). Values range between 1.0 (perfect separation of the test values of the two groups) and 0.5 (no apparent distributional difference between the two groups of test values). The area does not depend only on a particular portion of the plot such as the point closest to the diagonal or the sensitivity at 90% specificity, but on the entire plot. This is a quantitative, descriptive expression of how close the ROC plot is to the perfect one (area = 1.0).

When used herein, the term "non-rheumatic disorder" encompasses disorders causing musculoskeletal complaints which are preferably not caused by an autoimmune disorder. Preferably, said autoimmune disorder is characterized by the occurrence of autoantibodies against an autoantigen. It may also be characterized by the occurrence of antigens.

In the context of the present invention, a non-rheumatic disorder is preferably a non-autoimmune musculoskeletal disorder, i.e., it is not caused by an autoimmune disorder.

In the context of the present invention, a preferred non-rheumatic disorder is selected from the group consisting of osteoarthritis, osteoporosis, osteonecrosis, osteopenia, fibromyalgia, low back pain, (articular) gout, pseudogout, bursitis, trauma, fracture, tendinitis.

Osteoarthritis is a chronic arthropathy characterized by disruption and potential loss of joint cartilage along with other joint changes, including bone hypertrophy (osteophyte formation). Symptoms include gradually developing pain aggravated or triggered by activity, stiffness lasting < 30 min on awakening and after inactivity, and occasional joint swelling.

Osteoporosis is a progressive metabolic bone disease that decreases bone density (bone mass per unit volume), with deterioration of bone structure. Skeletal weakness leads to fractures with minor or inapparent trauma, particularly in the thoracic and lumbar spine, wrist, and hip. Acute or chronic back pain is common.

Osteonecrosis is a focal infarct of bone that may be caused by specific etiologic factors or may be idiopathic. It can cause pain, limitation of motion, joint collapse, and osteoarthritis.

Osteopenia is a condition where bone mineral density is lower than normal. It is considered to be a precursor to osteoporosis. However, not every person diagnosed with osteopenia will develop osteoporosis

Fibromyalgia is a common non-articular disorder of unknown cause characterized by generalized aching (sometimes severe), widespread tenderness of muscles, areas around tendon insertions, and adjacent soft tissues, as well as muscle stiffness, fatigue and poor sleep. In fibromyalgia, any fibromuscular tissues may be involved, especially those of the occiput, neck, shoulders, thorax, low back, and thighs.

Bursitis is acute or chronic inflammation of a bursa. The cause is usually unknown, but trauma, repetitive or acute, may contribute, as may infection and crystal-induced disease. Symptoms include pain (particularly with motion or pressure), swelling, and tenderness.

Tendinitis is inflammation of a tendon, often developing after degeneration (tendinopathy); tenosynovitis is tendinitis with inflammation of the tendon sheath lining. Symptoms usually include pain with motion and tenderness with palpation.

Gout is a painful and potentially disabling form of arthritis. Initial symptoms usually consist of intense episodes of painful swelling in single joints, most often in the feet (especially the big toe).

Pseudogout is a type of arthritis that, as the name implies, can cause symptoms similar to gout, but in reaction to a different type of crystal deposit. Pseudogout, sometimes referred to as calcium pyrophosphate deposition disease, can cause severe episodes of localized pain and swelling resulting in incapacitation for days or weeks. It also can cause more chronic arthritis that mimics osteoarthritis or rheumatoid arthritis. Knees are most often involved but wrists, shoulders, ankles, elbows or hands can be affected.

Low back pain is pain in the back. It can be localized to the lumbar spine or may radiate into the legs below the knee (sciatica).

A "musculoskeletal disorder (MSD)" is a condition where a part of the musculoskeletal system is traumatized, damaged and/or injured, thereby causing musculoskeletal complaints. MSD are also known as bone diseases, muscular diseases or diseases of the musculoskeletal system which terms may thus be interchangeably used herein.

The "musculoskeletal system" includes the body's bones (the skeleton), muscles, cartilage, tendons, ligaments, joints, connective tissue (the tissue that supports and binds tissues and organs together) including cartilage including articular cartilage (the smooth, glistening white tissue that covers the surface of all the diarthrodial joints in the body.

Accordingly, a musculoskeletal disorder also includes disorders of muscles, cartilage, tendons, ligaments, joints, connective tissue including cartilage including articular cartilage.

"Musculoskeletal complaints" have as chief symptom pain of a part of the musculoskeletal system. Typically, patients complain that their entire body aches. Other symptoms may be fatigue and/or sleep disturbances. A musculoskeletal complaint can be diagnosed by physical examination and medical history as well as by diagnostic through imaging or test assays such as the erythrocyte sedimentation rate (ESR).

"Pain" is the chief symptom of musculoskeletal complaint. The pain may be mild or severe, local or widespread (diffuse). Although pain may be acute and short-lived, as is the case with most injuries, pain may be ongoing with chronic illnesses. Pain may, for example, be deep, penetrating, or dull. Pain may range in intensity from slight through severe to agonizing and can, for example, appear as constant or intermittent. The threshold of pain may vary between individuals. Many attempts have been made to create a pain scale that can be used to quantify pain, for instance on a numeric scale that ranges from 0 to 10 points. In this scale, zero would be no pain at all and ten would be the worst pain imaginable. The purpose of these scales is to monitor an individual's pain over time, allowing care-givers to see how a patient responds to therapy for example.

Typically, MSDs affect the back, neck, shoulders and upper limbs; less often they affect the lower limbs. MSDs develop over time and are caused either by the work itself or by the employees' working environment. They can also result from accidents, e.g. fractures and dislocations. For example, MSDs may often be focused on a joint and affect the muscle and bone. However other areas can be strained and their response to that trauma can be an injury. Typically, MSDs affect the back, neck, shoulders and upper limbs; less often they affect the lower limbs.

Upper limb disorders (ULDs) affect the arms, from fingers to shoulder, and neck. They are often called repetitive strain injuries, 'RSI', cumulative trauma disorder or occupational overuse syndrome).ULDs include recognised conditions such as carpal tunnel syndrome, tenosynovitis, and also conditions where there is pain but no recognised condition can be identified. Other terms such as repetitive strain injury, cumulative trauma disorder or occupational overuse syndrome are used. Symptoms of upper limb disorder are tenderness, aches and pain, stiffness, weakness, tingling, numbness, cramp or swelling.

Back pain includes low back pain which can be extremely painful. It can be difficult to cope with the severe pain but fortunately it is rarely due to serious disease.

A "rheumatic disorder" is a disorder affecting the loco-motor system including joints, muscles, connective tissues, soft tissues around the joints and bones. It causes inflammation, swelling, and/or pain, for example, in the joints or muscles. In the context of the present invention, a rheumatic disorder is preferably characterized by an immune response against the body's own cells and tissues, in particular against the body's musculoskeletal system. Said rheumatic disorder is preferably chronic and/or is characterized by inflammation.

Accordingly, the rheumatic disorder is preferably an autoimmune musculoskeletal disorder, in particular a chronic, inflammatory autoimmune musculoskeletal disorder. Preferably, the autoimmune musculoskeletal disorder is a musculoskeletal disorder as described herein, whereby it is mediated by an autoimmune disorder which, apart from other symptoms, causes a musculoskeletal complaint. An autoimmune-mediated musculoskeletal disorder may thus affect the musculoskeletal system of the body. Autoimmune-mediated musculoskeletal disorders typically feature the presence and pathogenic role of circulating autoantibodies and/or autoreactive T cells.

The autoimmune musculoskeletal disorder is preferably one or more selected from rheumatoid arthritides, connective tissue diseases, and/or vasculitides as described herein. When used herein the term "arthritides" can be interchangeably used with the singular form "arthritis". Also, the term "vasculitides" when used herein can be interchangeably used with the singular form "vasculitis".

The term "rheumatic disorder" also includes as a group of disorder spondyloarthritides as well as infectious arthritides. Accordingly, it is envisaged by the present invention that the methods are also applied to test as to whether spondyloarthritides and/or infectious arthritides may be the cause of a musculoskeletal complaint. Specifically, in case of spondyloarthritides it is tested as to whether autoantibodies against the G1 domain of aggrecan are present in a sample obtained from a subject. Of course, the presence of any other autoantibodies against autoantigens that are indicative for spondyloarthritides, in particular psoriatic arthritis, reactive arthritis, enteropathic arthritis and/or undifferentiated spondoarthritis may be tested. In the alternative, an antigen indicative for spondyloarthritides may be detected by way of an antibody or by any known nucleic acid testing method.

For example, as described herein elsewhere, it may be tested as to whether the subject has the HLA-B27 MHC type (allele), since this allele is related to a high probability for the onset of Morbus Bechterew.

In case of infectious arthritides, the methods of the present invention can also be used. Accordingly, an antibody which is indicative for infectious arthritides recognizes streptolysin, an antigen derived from hepatitis virus B, hepatitis virus C antibody, parvovirus B19, Epstein-Barr virus, cytomegalovirus, Rubela virus and/or Arbovirus. Strictly spoken, in case of infectious arthritides the analyte to be determined is an antibody against an infectious agent causing Lyme disease arthritis, septic arthritis and/or viral arthritis. These conditions are cause by infectious agents such as hepatitis virus B, hepatitis virus C antibody, parvovirus B19, Epstein-Barr virus, cytomegalovirus, Rubela virus or Arbovirus.

Accordingly, the present invention envisages that antibodies which recognize streptolysin, an antigen derived from hepatitis virus B, hepatitis virus C, parvovirus B19, Epstein-Barr virus, cytomegalovirus, Rubela virus and/or Arbovirus are determined in a sample from a subject in order to exclude or include hepatitis virus B, hepatitis virus C antibody, parvovirus B19, Epstein-Barr virus, cytomegalovirus, Rubela virus and/or Arbovirus as cause of a musculoskeletal disorder. In the alternative, streptolysin, an antigen derived from hepatitis virus B, hepatitis virus C, parvovirus B19, Epstein-Barr virus, cytomegalovirus, Rubela virus and/or Arbovirus may be detected as an antigen indicative for infectious arthritides by way of an antibody or by any known nucleic acid testing method.

Thus, the methods of present invention further comprise determining as to whether at least one (auto)antibody or antigen which is indicative for at least one spondyloarthritides disorder and/or infectious arthritides disorder is present in a sample obtained from the subject.

The term "at least" when used together with the term "rheumatic disorder" includes that more than one, for example, 2, 3, 4, 5, 6, 7, 8, 9, 10, 11, 12, 13, 14, 15, 20, 25, 30, 35, 40, 45 or all rheumatic disorders may or, respectively, may not be determined due to the presence or absence, respectively, of at least one autoantibody which is indicative for said rheumatic disorder.

Accordingly, since it is known that one and the same autoantibody can be indicative for more than one rheumatic disorder, the autoantibody, the presence or absence of which is determined, may be indicative for more than one rheumatic disorder. For example, one and the same autoantibody may be indicative for 2, 3, 4, 5, 6, 7, 8, 9 or 10 or more rheumatic disoders, with 2, 3, 4 or 5 being preferred.

The rheumatoid arthritides preferably include rheumatoid arthritis and/or juvenile rheumatoid arthritis.

Rheumatoid arthritides denotes conditions characterized by an inflammation of the joints that is characterized by swelling, heat, and pain.

Rheumatoid arthritis (RA) is a chronic, systemic inflammatory disorder that may affect many tissues and organs, but primarily involves the joints, thereby producing an inflammatory synovitis that often progresses to destruction of the articular cartilage and ankylosis of the joints. It is characterized by severe joint pain due to inflammation and swelling. Rheumatoid arthritis can also produce diffuse inflammation in the lungs, pericardium, pleura, and sclera, and also nodular lesions, most common in subcutaneous tissue under the skin. Although the cause of rheumatoid arthritis is unknown, autoimmunity plays a pivotal role in its chronicity and progression.

Juvenile rheumatoid arthritis (juvenile idiopathic arthritis) is a form of rheumatoid arthritis which usually affects kids under age 16. Arthritis, fever, rash, adenopathy, splenomegaly, and iridocyclitis are typical of Juvenile rheumatoid arthritis.

The spondyloarthritides preferably include psoriatic arthritis, ancylosing spondylitis (Morbus Bechterew), reactive arthritis, enteropathic arthritis and/or undifferentiated spondoarthritis.

Spondyloarthritides (or spondyloarthropathies) is the overall name for a family of inflammatory rheumatic diseases that can affect the spine and joints, ligaments and tendons. These diseases can cause fatigue and pain or stiffness in the back, neck, hands, knees, and ankles as well as inflammation of the eyes, skin, lungs, and heart valves.

Specifically, the term "spondyloarthritides" thus covers a group of closely related inflammatory diseases including arthritis of the spine (sacroiliitis or spondylitis) and peripheral joints; as well as inflammation in the area where ligaments and tendons attach to bones (enthesitis or enthesopathy). These diseases can cause pain in the spine, legs and arms as joints, ligaments, and tendons become inflamed and/or predispose patients to spinal vertebral fractures. Skin rashes, eye, and intestinal problems can also occur.

Diseases that fall under spondyloarthritis umbrella can include: 1) ankylosing spondylitis; 2) reactive arthritis (known previously as Reiter's syndrome) 3) psoriatic arthritis and psoriatic spondylitis, and 4) the arthritis or spondylitis associated with the inflammatory bowel diseases, ulcerative colitis and Crohn's disease, i.e., enteropathic arthrirtis. Still other patients may develop undifferentiated spondyloarthritis. This means they have symptoms or signs of one of the illnesses above, but do not necessarily develop the full blown disease. Examples of disorders belonging to undifferentiated spondyloarthritis are Whipple disease and Behçet disease.

Psoriatic arthritis is a type of arthritic inflammation that occurs in patients who have a skin rash called psoriasis. This particular arthritis can affect any joint within the body, with symptoms that vary from person to person. It is known that persistent inflammation from psoriatic arthritis can lead to joint damage.

Psoriatic arthritis can affect any joint within the body, either in a single joint or in the same joint on both sides of the body, e.g., one or both knees. Affected fingers and toes get thickened, a condition often referred to as dactylitis.

Psoriatic arthritis in the spine, called spondylitis, causes pain in the back or neck, and difficulty bending. It can also cause tender spots at sites in the body where tendons and ligaments join onto bones. This condition, called enthesopathy, can result in pain at the back of the heel, the sole of the foot, or other areas.

Ankylosing spondylitis (AS, from Greek ankylos, bent; spondylos, vertebrae), also known as Bechterew's disease, Bechterew syndrome, and Marie Strümpell disease, a form of spondyloarthritis, is a chronic, inflammatory arthritis and autoimmune disease. It mainly affects joints in the spine and the sacroilium in the pelvis, causing eventual fusion of the spine. For many with AS, but more so for women, in addition to the spine the disease can affect other joints throughout the body such as the shoulders, hips, knees, feet, as well as other joints. For some, peripheral joints are affected before there is ever any spine pain or inflammation.

Reactive arthritis is a form of arthritis that develops in reaction to an infectious agent, such as bacteria, usually in the bowel or genital areas. Those developing the illness experience pain, swelling and stiffness, usually in larger joints, particularly the lower limbs, as well as the spine and ligaments. Typically the immune system readjusts its response to the infection and the condition may ultimately go away.

Although many infections, including viruses, can result in a reactive arthritis, the term usually applies to symptoms occurring after an infection of the bowel (caused by the germs Campylobacter, Salmonella, Shigella and Yersinia) or genitals (caused by *Chlamydia trachomatis).*

Enteropathic arthritis involves the peripheral joints, usually in the lower extremities such as the knees or ankles. It commonly involves only a few or a limited number of joints and may closely follow the bowel condition. The synovitis is generally self-limited and non-deforming.

Undifferentiated spondyloarthropathy (or spondoarthritis) is a term used to describe symptoms and signs of spondylitis in someone who does not meet the criteria for a definitive diagnosis of AS or related disease. Although unrecognized by many physicians, sometimes a doctor may make an initial diagnosis of "Spondyloarthropathy" or "Unclassified Spondyloarthropathy" if certain symptoms are present but are not enough to make a specific diagnosis. Over time, some people with undifferentiated spondyloarthropathy will develop a well-defined form of spondylitis such as ankylosing spondylitis

The connective tissue diseases preferably include systemic lupus erythematosus, systemic sclerosis, polymyositis, dermatomyositis, sjögren syndrome, CREST-syndrome and/or mixed connective tissue disease.

A connective tissue disease (also known as collagenosis) is a disease that has the connective tissues of the body as a target of pathology. Connective tissue is any type of biological tissue with an extensive extracellular matrix that supports, binds together, and protects organs. These tissues form a framework, or matrix, for the body, and are composed of two major structural protein molecules, collagen and elastin. There are many different types of collagen protein in each of the body's tissues. Elastin has the capability of stretching and returning to its original length - like a spring or rubber band. Elastin is the major component of ligaments (tissues that attach bone to bone) and skin. In patients with connective tissue disease, it is common for collagen and elastin to become injured by inflammation. Many connective tissue diseases feature abnormal immune system activity with inflammation in tissues as a result of an immune system that is directed against one's own body tissues (autoimmunity).

Systemic lupus erythematosus (SLE) is characterized by an inflammation of connective tissues with variable features frequently including fever, weakness, joint pains or arthritis resembling rheumatoid arthritis, diffuse erythematosus skin lesions on the face, neck and upper extremities, degeneration of the basal layer and epidermal atrophy and other evidence of an autoimmune phenomenon. Usually, patients have skin rashes and arthritis, as well as fatigue and fever.

Sj6gren's syndrome (or sjögren/sjogren syndrome) is an inflammatory disease that can affect many different parts of the body, but most often affects the tear and saliva glands. Patients with this condition may notice irritation, a gritty feeling, or painful burning in the eyes. Dry mouth or difficulty eating dry foods, and swelling of the glands around the face and neck are also common. Some patients experience dryness of other mucous membranes (such as the nasal passages, throat, and vagina) and skin. "Primary" Sjögren's syndrome occurs in people with no other rheumatologic disease. "Secondary" Sjögren's occurs in people who do have another rheumatologic disease, most often lupus and rheumatoid arthritis.

Most of the complications of Sjögren's syndrome occur because of decreased tears and saliva. Patients with dry eyes are at increased risk for infections around the eye and may have damage to the cornea. Dry mouth may cause an increase in dental decay, gingivitis (gum inflammation), and oral yeast infections (thrush) that may cause pain and burning. Some patients have episodes of painful swelling in the saliva glands around the face. Pain and stiffness in the joints with mild swelling may occur in some patients, even in those without rheumatoid arthritis or lupus. Rashes on the arms and legs related to inflammation in small blood vessels (vasculitis) and inflammation in the lungs, liver, and kidney may occur rarely and be difficult to diagnose. Neurological complications that cause symptoms such as numbness, tingling, and weakness have also been described in some patients.

Systemic sclerosis (scleroderma) is a rare chronic disease of unknown cause characterized by diffuse fibrosis, degenerative changes, and vascular abnormalities in the skin, joints, and internal organs (especially the esophagus, lower Gl tract, lung, heart, and kidney). Common symptoms include Raynaud's syndrome, polyarthralgia, dysphagia, heartburn, and swelling and eventually skin tightening and contractures of the fingers. Lung, heart, and kidney involvement accounts for most deaths.

Systemic sclerosis varies in severity and progression, ranging from generalized skin thickening with rapidly progressive and often fatal visceral involvement (Systemic sclerosis with diffuse scleroderma) to isolated skin involvement (often just the fingers and face) and slow progression (often several decades) before visceral disease develops. The latter form is termed limited cutaneous scleroderma or CREST syndrome (Calcinosis cutis, Raynaud's syndrome, Esophageal dysmotility, Sclerodactyly, Telangiectasias). In addition, Systemic sclerosis can overlap with other autoimmune rheumatic disorders - e.g., sclerodermatomyositis (tight skin and muscle weakness indistinguishable from polymyositis) and mixed connective tissue disease.

Localized scleroderma, including deep and extensive lesions, can prevent normal motion of joints and interfere with daily activities.

Polymyositis and dermatomyositis are uncommon systemic rheumatic diseases characterized by inflammatory and degenerative changes in the muscles (polymyositis) or in the skin and muscles (dermatomyositis). The most specific skin signs are Gottron's papules over the knuckles and a periorbital heliotropic rash. Manifestations include symmetric weakness, some tenderness, and later atrophy, principally of the proximal limb girdle muscles.

Mixed connective tissue disease is an uncommon, specifically defined, overlap syndrome characterized by clinical features of SLE, systemic sclerosis, and polymyositis with very high titers of circulating antinuclear antibody to a ribonucleoprotein antigen. Hand swelling, Raynaud's syndrome, polyarthralgia, inflammatory myopathy, esophageal hypomotility, and pulmonary dysfunction are common.

The vasculitides preferably include polymyalgia rheumatica, giant cell artheritis, Morbus Wegener and/or Behcet's syndrome.

Vasculitides encompass conditions characterized by inflammation of blood vessels, often with ischemia, necrosis, and occlusive changes. It can affect any blood vessel—arteries, arterioles, veins, venules, or capillaries. Most damage results when inflammation narrows vessels and causes tissue necrosis. Clinical manifestations of specific vasculitic disorders are diverse and depend on the size of the involved vessels and the organs affected by ischemia.

Polymyalgia rheumatica (PMR) is a syndrome closely associated with giant cell (temporal) arteritis. It typically affects adults older than 55. It typically causes severe pain and stiffness in proximal muscles, without weakness or atrophy, and nonspecific systemic symptoms. Polymyalgia rheumatica is characterized by bilateral proximal aching of the shoulder and hip girdle muscles and the back (upper and lower) and neck muscles. Stiffness in the morning is typical. Shoulder symptoms may reflect proximal bursitis (eg, subdeltoid, subacromial) and less often bicipital tenosynovitis or joint synovitis. Discomfort is worse in the morning and is occasionally severe enough to prevent patients from getting out of bed and from doing simple activities.

Giant cell arteritis (GCA) is a type of vasculitis or arteritis, a group of diseases whose typical feature is inflammation of blood vessels. In the case of GCA, the vessels most commonly involved are the arteries of the scalp and head, especially the arteries over the temples, which is why another term for GCA is "temporal arteritis."

GCA can overlap with another disease, polymyalgia rheumatica. At some point, 5 to 15% of patients with PMR will be diagnosed with GCA. Looked at another way, about 50% of patients with GCA have symptoms of PMR. The symptoms of the two conditions can occur at the same time or separately.

The most common symptom of GCA is a new headache, usually in the area of the temples, although headache due to GCA can occur anywhere, including the front, top and back of the skull. Almost as common are more generalized symptoms, such as unusual fatigue, loss of appetite, weight loss, a flu-like feeling or fevers. Occasionally the only indication of GCA is a recurring, prolonged fever. Less common symptoms involve pain in the jaw when chewing or facial, tongue or throat pain.

Wegener's granulomatosis (Morbus Wegener) is characterized by necrotizing granulomatous inflammation, small and medium-sized vessel vasculitis, and focal necrotizing glomerulonephritis, often with crescent formation. Typically, the upper and lower respiratory tract and the kidneys are affected, but any organ may be. Symptoms vary depending on the organs and systems affected. Patients may present with upper and lower respiratory tract symptoms (e.g., recurrent nasal discharge or epistaxis, cough), followed by hypertension and edema, or with symptoms reflecting multiorgan involvement. Other symptoms can include joint pain and numbness or loss of movement in the fingers, toes or limbs.

Behçet's syndrome is a multisystem, relapsing, chronic vasculitic disorder with prominent mucosal inflammation. Common manifestations include recurrent oral ulcers, ocular inflammation, genital ulcers, and skin lesions. The most serious manifestations are blindness, neurologic or Gl manifestations, venous thromboses, and arterial aneurysms. Diagnosis is clinical, using international criteria.

As far as musculoskeletal symptoms are concerned that are usually relatively mild, self-limiting, and nondestructive arthralgias or frank arthritis, especially in the knees and other large joints, occur in about 50% of patients. Sacroiliac inflammation can occur.

The infectious arthritides preferably include Lyme disease arthritis, septic arthritis and/or viral arthritis.

Infectious arthritis (septic arthritis) is infection in the fluid and tissues of a joint usually caused by bacteria, but sometimes caused by viruses or fungi. Bacteria or sometimes viruses or fungi may spread through the bloodstream or from nearby infected tissue to a joint, causing infection. Pain, swelling, and fever of joints and muscles combined with fatigue usually develop within hours or a couple of days.

Infecting organisms, mainly bacteria, usually reach the joint through the bloodstream, but a joint can be infected directly if it is contaminated during surgery or by an injection or an injury. Different bacteria can infect a joint, but the bacteria most likely to cause infection depend on a person's age. Staphylococci and bacteria known as gram-negative bacilli most often infect infants and young children, whereas gonococci, staphylococci, and streptococci most often infect older children and adults. Occasionally, spirochetes, such as those that cause Lyme disease and syphilis, can infect joints.

Viruses such as the human immunodeficiency virus (HIV); parvoviruses, and those that cause rubella, mumps, and hepatitis C or B can infect joints in people of any age. A slowly developing chronic infectious arthritis is most often caused by *Mycobacterium tuberculosis* or fungi.

Lyme disease is an infection caused by the bacteria, *Borrelia burgdorferi,* which enters the body when certain ticks bite. In its early localized stage, a skin rash, called erythema migrans, appears at the location of the tick bite from three days to several weeks later. The rash starts as a small red mark and gradually grows in width to at least two inches.

"Antibody" refers to a polypeptide ligand substantially encoded by an immunoglobulin gene or immunoglobulin genes, or fragments thereof, which specifically binds and recognizes an epitope (e.g., an antigen). The recognized immunoglobulin genes include the kappa and lambda light chain constant region genes, the alpha, gamma, delta, epsilon and mu heavy chain constant region genes, and the myriad immunoglobulin variable region genes. Antibodies exist, e.g., as intact immunoglobulins or as a number of well characterized fragments produced by digestion with various peptidases. This includes, e.g., Fab' and F(ab)'2 fragments. The term "antibody," as used herein, also includes antibody fragments either produced by the modification of whole antibodies or those synthesized de novo using recombinant DNA methodologies. It also includes polyclonal antibodies, monoclonal antibodies, chimeric antibodies, humanized antibodies, or single chain antibodies. "Fc" portion of an antibody refers to that portion of an immunoglobulin heavy chain that comprises one or more heavy chain constant region domains, CH1, CH2 and CH3, but does not include the heavy chain variable region.

An "autoantibody" applied in the present invention is an antibody produced by the immune system of a subject that is directed against one or more of the subject's own proteins (self-antigens or autoantigens). Preferably, the autoantibody applied in the embodiments of the present invention is indicative for rheumatoid arthritides, connective tissue diseases and/or vasculitides as described herein.

The term "indicative for" when used in the context of an autoantibody applied in the embodiments of the present invention includes that an autoantibody, the presence or absence of which is determined in the embodiments of the present invention, is typically present in a subject having a rheumatic disorder. "Typically present" means that the autoantibody is usually associated with a rheumatic disorder. "Usually associated" includes a probability of more than 50%, preferably more than 60%, more preferably more than 70%, even more preferably more than 80% and particularly preferred more than 90 or 95%.

The rheumatic disorder is most preferably rheumatoid arthritides, connective tissue diseases, and/or vasculitides.

The term "indicative for" also includes that the autoantibody, the presence or absence of which is determined in the embodiments of the present invention is directed against, binds to, reacts with or recognizes autoantigens, i.e., self-antigens of the subject that produced them. For example, by binding to, reacting with or recognizing an autoantigen the presence of an autoantibody in a sample obtained from a subject can be determined. Likewise, by not binding to, not reacting with or not recognizing an autoantigen the absence of an autoantibody in a sample obtained from a subject can be determined.

The term "indicative for" also includes that antigens, the presence or absence of which is determined in some of the embodiments of the present invention is bound by an antibody directed against, reacts with or recognizes said antigens that may occur in a subject having a rheumatic disorder.

Accordingly, in an alternative aspect the present invention relates to a method for excluding a non-rheumatic disorder as cause of a musculoskeletal complaint of a subject comprising determining the presence of at least one autoantibody or antigen by binding to or reacting with an autoantigen or antibody which is indicative for at least one rheumatic disorder in a sample obtained from the subject, wherein the binding of an autoantibody to an autoantigen or the reaction of an antigen with an antibody allows to exclude a non-rheumatic disorder as cause of a musculoskeletal complaint of a subject.

Likewise, in another alterative and/or additional aspect the present invention relates to a method for excluding a rheumatic disorder as cause of a musculoskeletal complaint of a subject comprising determining the absence of at least one autoantibody or antigen by not binding to or not reacting with an autoantigen or antibody which is indicative for at least one rheumatic disorder in a sample obtained from the subject, wherein the non-binding of an autoantibody to an autoantigen or the non-reaction of an antigen with an antibody allows to exclude a rheumatic disorder as cause of a musculoskeletal complaint of a subject.

An "antigen" is any molecule that binds, preferably specifically, to an antibody. It also includes any molecule that can be bound by an MHC molecule and presented to a T-cell receptor. In the present invention an antigen is preferably an antigen that occurs in a subject having a rheumatic disorder.

"Autoantigen" is used to denote antigens for which the presence or absence of antibodies in a sample of a subject is determined. An "autoantigen" in the sense of the present invention is an endogenous tissue constituent that has the ability to interact with autoantibodies and may cause an immune response, thereby causing, inter alia, a musculoskeletal complaint.

An autoantigen or antigen can be an entire mature form of a protein, such as a protein referred to as a target antigen or can be a precursor, processed form, unprocessed form, isoforms, variant, a fragment thereof that includes an epitope, or allelic variant thereof, providing that the modified, processed, or variant for of the protein has the ability to bind autoantigens present in samples from subjects. It can be isolated from a subject, recombinantly or synthetically produced.

Autoantigens are contemplated to include any fragments thereof, in particular, immunologically detectable fragments. They are also meant to include immunologically detectable products of proteolysis of the proteins, as well as processed forms, post-translationally modified forms, such as, for example, "prep" "pro," or "prepro" forms of markers, or the "prep" "pro," or "prepro" fragment removed to form the mature protein, as well as allelic variants and splice variants of the autoantigens. An "autoantigen" refers to a molecule, such as a protein, endogenous to the host that is recognized by an autoantibody. The autoantigen may, for example, also be a complex of proteins, DNA or RNA, either single- or double-stranded or a glycoprotein.

As used herein, the term "peptide," "oligopeptide," and "polypeptide" are used interchangeably with protein herein and refer to a sequence of contiguous amino acids linked by peptide bonds. As used herein, the term "protein" refers to a polypeptide that can also include post-translational modifications that include the modification of amino acids of the protein and may include the addition of chemical groups or biomolecules that are not amino acid-based. The terms apply to amino acid polymers in which one or more amino acid residue is an analog or mimetic of a corresponding naturally occurring amino acid, as well as to naturally occurring amino acid polymers. Polypeptides can be modified, e.g., by the addition of carbohydrate residues to form glycoproteins. The terms "polypeptide," "peptide" and "protein" include glycoproteins, as well as non-glycoproteins.

A "variant" (for example, an allelic variant or splice variant) of an autoantigen, as used herein, refers to an amino acid sequence that is altered with respect to the referenced polypeptide or protein by one or more amino acids. In the present invention, a variant of an autoantigen retains the antigenicity, or antibody-binding property, of the referenced autoantigen. In preferred aspects of the invention, a variant of an autoantigen can be bound by the same population of autoantibodies that are able to bind the referenced autoantigen. Preferably a variant of an autoantigen has at least 60% identity to the referenced autoantigen over a sequence of at least 15 amino acids. More preferably a variant of an autoantigen is at least 70% identical to the referenced autoantigen over a sequence of at least 15 amino acids. Autoantigen variants can be, for example, at least 80%, at least 90%, at least 95%, or at least 99% identical to referenced autoantigen over a sequence of at least 15 amino acids. Autoantigen variants of the invention can be, for example, at least 80%, at least 90%, at least 95%, or at least 99% identical to referenced autoantigen over a sequence of at least 20 amino acids. The variant may have "conservative" changes, wherein a substituted amino acid has similar structural or chemical properties (e.g., replacement of leucine with isoleucine). A variant may also have "non-conservative" changes (e.g., replacement of glycine with tryptophan). Analogous minor variations may also include amino acid deletions or insertions, or both. Guidance in determining which amino acid residues may be substituted, inserted, or deleted without abolishing immunological reactivity may be found using computer programs well known in the art.

A fragment of an autoantigen that includes an epitope recognized by an autoantibody can be at least 5, 6, 7, 8, 9, 10, 11, 12, 13, 14, 15, 20, 25, 30, 35, 40, 45, 50, 75, 100, 150, 200, 250, 300, 350, 400, 450, 500, 750, or 1000 amino acids in length. The fragment can also be between 5, 10, 15, 20, 25, 50, 75, 100, 150, 200, or 250 and one amino acid less than the entire length of an autoantigen. Typically, such epitopes are characterized in advance such that it is known that autoantibodies for a given autoantigen recognize the epitope. Methods for epitope mapping are well known in the art. An "epitope" is a site on an antigen, such as an autoantigen disclosed herein, recognized by an autoantibody.

The autoantigen in the sense of the present invention is preferably at least one associated with a rheumatic disorder. The rheumatic disorder is most preferably rheumatoid arthritides, connective tissue diseases and/or vasculitides.

Normally autoantibodies are routinely eliminated by the immune system's self-regulatory process -probably through the neutralization of autoantibody-producing lymphocytes before they mature. However, at times this process fails, and antibodies that react to self constituents proliferate. Generally, autoantibodies damage body tissues by bringing about the phagocytosis (ingestion) or lysis (bursting) of healthy cells. Autoantibodies also interfere with the normal functioning of cells. Moreover, by attacking the subject's own cells, tissues, and/or organs, autoantibodies cause, for example, inflammation and/or damage. The causes of autoantibody production are varied and not well understood.

Being directed to, binding to, reacting with or recognizes means that the autoantibody applied in the embodiments of the present invention specifically binds to autoantigens as described herein. The term "specifically" in this context means that the autoantibody reacts with its counterpart autoantigen but essentially not with another protein. A "counterpart autoantigen" is an autoantigen which is bound by an autoantibody.

For example, an autoantibody directed against CCP (a typical autoantigen indicative for RA) does preferably not essentially bind to a protein which is closely related to CCP. Accordingly, the term "another protein" includes any protein including proteins closely related to or being homologous to a respective autoantigen against which the autoantibody is directed to. The term "does not essentially bind" means that the autoantibody applied in the embodiments of the present invention does not bind another protein, i.e., shows a cross-reactivity of less than 30%, preferably 20%, more preferably 10%, particularly preferably less than 9, 8, 7, 6, 5, 4, 3, 2, 1 %.

Whether the autoantibody specifically reacts as defined herein above can easily be tested, inter alia, by comparing the reaction of said autoantibody with its counterpart autoantigen with the reaction of said autoantibody with (an)other protein(s).

The term "at least one autoantibody" when used in the context of the present invention includes that the presence or absence of more than one autoantibody is determined. For example, the presence or absence of a panel of autoantibodies which is indicative for rheumatoid arthritides, connective tissue diseases and/or vasculitides may be determined.

A panel may include at least 1, 2, 3, 4, 5, 6, 7, 8, 9, 10, 11, 12, 13, 14, 15, 16, 17, 18, 19, 20,21,22,23,24,25,26,27,28,29,30,31,32,33,34,35,40,45,50,55,60,65,70, 75, 80, 85, 90, 95, 100, 120, 140, 150, 160, 180, 200, 220, 240, 250, 260, 280, 300, 400, 500, 600, 700, 800, 900 or 1000 autoantibodies or antigens.

Thus, the presence or absence of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 40, 45, 50, 55, 60, 65, 70, 75, 80, 85, 90, 95, 100, 120, 140, 150, 160, 180, 200, 220, 240, 250, 260, 280, 300, 400, 500, 600, 700, 800, 900 or 1000 autoantibodies or antigens which are indicative for a rheumatic disorder may be determined.

For example, when determining the presence or absence of an autoantibody, the presence or absence of at least 1, 2, 3, 4, 5, 6, 7, 8, 9, 10, 11, 12, 13, 14, 15, 16, 17, 18, 19,20,21,22,23,24,25,26,27,28,29,30,31,32,33,34,35,40,45,50,55,60,65, 70, 75, 80, 85, 90, 95, 100, 120, 140, 150, 160, 180, 200, 220, 240, 250, 260, 280, 300, 400, 500, 600, 700, 800, 900 or 1000 autoantigens which are indicative for a rheumatic disorder is determined.

As described above, the autoantibodies, the presence or absence of which is determined in the embodiments of the present invention, are most preferably indicative for rheumatoid arthritides, connective tissue diseases and/or vasculitides.

The autoantibody which is indicative for rheumatoid arthritides recognizes preferably CCP, ANA and/or RF.

Citrullinated peptides (CP) including cyclic citrullinated peptides (CCP), which are preferably applied in the methods, uses and kits of the present invention, are antigens for rather important autoantibodies as found in the sera of patients with RA. They have been intensively studied during the past years by several groups of researchers (cf. e.g., WO 98/08946; WO 98/22503; WO 99/28344; WO 99/35167, WO 01/46222, and WO 03/050542). Recently Schellekens and co-workers (Schellekens, G. A. et al., Arthritis Rheum. 43 (2000) 155-163) reported that an ELISA-test based on specific cyclic citrullinated peptides (CCP) showed superior performance characteristics with regard to diagnostic accuracy for RA as compared to the same assay using linear peptides. Autoantibodies against CCP, i.e., antibodies which most likely are reactive with citrullinated polypeptides circulating in a patient serum and which bind to CCP in an in vitro assay are termed "anti-CCP"; see also WO 2007/039280.

Rheumatoid factor (RF) is an antibody against the Fc portion of lgG, which is itself an antibody. RF and lgG join to form immune complexes which contribute to the disease process. Not all people with rheumatoid arthritis have detectable rheumatoid factor. Those who do not are said to be "seronegative". Rheumatoid factor can also be a cryoglobulin (antibody that precipitates on cooling of a blood sample); it can be either type 2 (monoclonal lgM to polyclonal lgG) or 3 (polyclonal lgM to polyclonal lgG) cryoglobulin.

The autoantibodies which are indicative for connective tissue diseases recognize preferably the antinuclear antibodies (ANA).

As the term "ANA (=antinuclear antibodies)", indicates, ANA are directed against a variety of nuclear antigens and have been detected in the serum of patients with many rheumatic and non-rheumatic diseases, as well as in patients with no definable clinical syndrome. Evidence-based guidelines for the use of immunological assays detecting ANA are available (Solomon D.H., Arthritis and Rheumatism 47 (2002) 434-444).

It is now, for example, possible to include individual highly purified antigens for the various antinuclear antibodies of different specificity and to thus specifically detect the corresponding ANA. For example antibodies to double-stranded DNA, or to antigens termed SSA, SSB, Sm/RNP, centromer, etc, can now be specifically detected.

Other ANA-antigens include the antigens to SSA60 (or Ro/SSA60), SSA52 (or Ro/SSA52), SSB (or La), Jo-l, Sc170, Sm/RNP, double-stranded DNA, and centromer B peptide (CENB-P).

In the diagnosis of a connective tissue diseases a positive value for at least one of the autoantigens SSA60, SSA52, SSB, Jo-l, Scl70, Sm/RNP, double- stranded DNA, and centromer B peptide is considered as ANA-positive.

Several of the above autoantigens are described in some detail below:

### SSA OR Ro(SSA)ANTIGEN:

Autoantibodies to the Ro(SSA) antigen are one of the most frequent serological markers of autoimmunity in rheumatic diseases (Tan, E.M., Adv. Immunol. 44 (1989) 93-151; McCauliffe, D.P. and Sontheimer, R.D., J. Inv. Dermatol. 100 (1993) 73S-79S). They are present in the serum of 50-80% of patients with Sjogren's syndrome (SS), 30-40% of patients with systemic lupus erythematosus (SLE), and 3-5% of patients with rheumatoid arthritis (RA) (Harley, J.B. et al., Arthritis Rheum. 29 (1986) 196-206; Reichlin, M., J. Clin. Immunol. 6 (1986) 339- 348). Ro(SSA) antibodies target protein antigens associated with small RNA molecules known as hY-RNAs. These protein-RNA complexes are referred to as Ro-ribonucleoproteins (Ro-RNPs) including RNP68, RNPA, RNPC.

Anti-Ro(SSA) positive sera may contain two different types of autoantibody; those directed to a 60 kDa and those directed to a 52 kDa polypeptide component (referred to as Ro60 and Ro52, or SSA60 and SA52, respectively) (Chan, E.K.L. and Buyon, J.P., Man. Biol. Markers Dis. (Kluwer Acad. Publ.) (1994) B4.1/1-18). While the vast majority of Ro(SSA) positive sera react with both of these components, anti-Ro60 antibody has been reported to occur without anti-Ro52 antibody only in SLE sera (Ben-Chetrit, E. et al., Arthritis Rheum. 33 (1990) 349-355; Slobbe, R.L. et al., Clin. Exp. Immunol. 86 (1991 ) 99-105) whereas anti-Ro52 antibody has been reported to occur in the absence of anti-Ro60 antibody in idiopathic inflammatory myopathy, dermatomyositis and scleroderma (Frank, M. B. et al., J. Autoimmun. 12 (1999) 137-142; Peene, l. et al., Ann. Rheum. Dis. 61 (2002) 1090-1094). Ro52 is a member of the tripartite motif (TRIM) family of proteins. The TRIM motif includes three zinc-binding domains, a RING finger, a B-box type 1 and a B-box type 2, and a centre coiled-coil region (leucine zipper). TRIM proteins are believed to identify specific cell compartments through a process of homomultimerisation (Reymond, A. et al., EMBO J. 20 (2001) 2140-2151).

### SSB OR LA(SSB) ANTIGEN:

Autoantibodies to the La (SSB) antigen can be detected in the sera of up to 87% of patients with primary or secondary SS. The presence of anti-La (SSB) autoantibodies usually coincides with the presence of anti-Ro (SSA) autoantibodies, however the fact that anti-Ro autoantibodies are far more common in other rheumatological conditions such as systemic lupus erythematosus (SLE) and mixed connective tissue disease (MCTD) suggests that anti-La is more specific for primary and secondary SS than anti-Ro (St. Clair, E.W., Rheum. Dis. Clin. N. America 18 (1992) 359-376; Harley, J.B., J. Autoimmun. 2 (1989) 383-394). The La (SSB) antigen binds to the oligo(U) 3' termini of nascent RNA polymerase III transcripts and facilitates transcriptional termination and reinitiation by this enzyme (Stefano, J.E., Cell 36 (1984) 145-154; Gottlieb, E. and Steitz, J.A., EMBO J. 8 (1989) 841- 850). It has also been reported to function as an ATP-dependent helicase able to melt RNA-DNA hybrids (Bachmann, M. et al., Cell 60 (1990) 85-93)

### J01 ANTIGEN:

The most common autoantibody in Polymyosytosis and Dermatomyositosis is anti-Jo-1, occurring in 15-20% of all myositosis patients and about 30% of adult PM patients (Nishikai, M. and Reichlin, M., Arthritis Rheum. 23 (1980) 881-888; Targoff, I.N., Rheum. Dis. Clin. North Am. 18 (1992) 455-482). In 1983 the Jo-l antigen was reported by Mathews and Bernstein (Mathews, M. B. and Bernstein, R.M., Nature 304 (1983) 177-179) to be histidyl-tRNA synthetase (HRS), an enzyme which catalyses the coupling of histidine to its specific tRNA before transport to the ribosome followed by incorporation into a polypeptide chain during protein synthesis.

### RNP/SM ANTIGENS:

Autoantibodies directed against the snRNP (small nuclear ribonucleoprotein) autoantigens referred to as RNP (including RNPA, RNPB and RNP68) and Sm (including SmB and SmD) were originally detected in the sera of systemic lupus erythematosus (SLE) patients (Tan, E.M. and Kunkel, H. G., J. Immunol. 96 (1966) 464-471; Mattioli, M. and Reichlin, M., J. Immunol. 107 (1971) 1281-1290). Anti-RNP antibodies were subsequently found in the sera of mixed connective tissue disease (MCTD) patients (Sharp, G. C. et al., Am. J. Med. 52 (1972) 148-159).

The snRNPs are a group of nuclear particles comprised of several polypeptides associated with a small nuclear RNA molecule. The most abundant snRNPs are involved in premRNA-splicing (Luehrmann, R. et al., Biochim. Biophys. Acta 1087 (1990) 265-292). A snRNP which is preferably applied in the embodiments of the present invention is U1-70, sometimes also referred to as U1 snRNP 70.

### SCL70 ANTIGEN:

Approximately 20-28% of scleroderma patients have autoantibodies to a nuclear protein referred to as Scl-70 (Douvas, A.S. et al., J. Biol. Chem. 254 (1979) 10514- 10522). In 1986 the Scl-70 antigen was identified by Shero et al. to be the superhelical DNA-relaxing enzyme topoisomerase I (Shero, J. H. et al., Science 231 (1986) 737-740).

### DOUBLE-STRANDED DNA (DSDNA):

Antibodies to double-stranded DNA are rarely seen in healthy individuals and considered a hallmark of systemic lupus erythematosus (SLE). They are usually determined by a fluorescent assay based on a DNA-containing organelle of Crithidia lucilliae, by radio immunoassay (RIA) or ELISA. It has been reported that between 50 and 90% of untreated SLE patients test positive for anti-dsDNA antibodies (Griesmacher, A. and Peichl P., Clin. Chem. Lab. Med. 39 (2001) 189- 208). Clinical improvements in patients with SLE are often associated with a decrease or a complete disappearance of anti-dsDNA antibodies.

### CENTROMERE B-PEPTIDE:

Anti-centromere antibodies (ACA) are found in 80-90% of patients with the limited cutaneous (CREST) variant of systemic sclerosis. They are directed to restricted regions of chromosomes (Barland, P. and Lipstein, E., Am. J. Med. 100 (1996) 16S-23S). ELISA methods based on recombinant CENP-B peptide are reported to be more sensitive in identifying ACA than immunofluorescence assays (Parveen, S., et al., J. Gasteroenterol. Hepatol. 10 (1995) 438-445).

Since most of the individual autoantibodies discussed above are to a certain extend indicative for a specific subgroup of autoimmune patients, such values for one or more specific ANA will preferably be included into an evaluation algorithm in order to indicate to which sub-group of rheumatic diseases other than RA such samples will likely belong to.

In a further preferred embodiment according to the present invention the testing for anti-CCP-antibodies and the testing for ANA are performed from the same sample in one single assay procedure by employing a protein chip. In such protein chip the antigen for measurement of anti-CCP as well as the various antigens for measurement for ANA are coated onto a solid support in individual areas, the autoantibodies in the sample are bind to their corresponding autoantigen and all autoantibodies bound are detected as described in US 6,815,217.

The ideal scenario for diagnosis would be a situation wherein a single event or process would cause the respective disease as, e.g., in infectious diseases. In all other cases correct diagnosis can be very difficult, especially when the etiology of the disease is not fully understood as is the case for RA. Therefore, generally various clinical symptoms and biological markers are considered together for diagnosis of RA. Markers can either be determined individually or in a preferred embodiment of the invention they can be measured simultaneously using a chip or a bead based array technology. The concentrations of the biomarkers are then interpreted independently using an individual cut-off for each marker or they are combined for interpretation.

In a method according to the present invention at least the concentration of the biomarkers anti-CCP and ANA, respectively, is determined and the marker combination is correlated to the absence or presence of rheumatoid arthritis, wherein in a preferred mode of correlating the measurements for anti-CCP and ANA, samples positive for anti-CCP and negative for ANA are considered indicative for RA. Samples fulfilling the requirement of testing positive for anti-CCP and negative for ANA are very likely to come from a patient with RA.

For RA, in addition to the presence of autoantibodies against CCP, RF and/or ANA, the amount of a biomarker such as CRP or IL-6 can be determined. The determination can be accomplished by any immunoassay as described herein and/or by any nucleic acid based technique such as PCR.

C-reactive protein (CRP) is a homopentameric Ca2+-binding acute phase protein with 21 kDa subunits that is involved in host defense. CRP synthesis is induced by IL-6, and indirectly by IL-I, since IL-I can trigger the synthesis of IL-6 by Kupffer cells in the hepatic sinusoids. The normal plasma concentration of CRP is < 3µg/ml (30 nM) in 90% of the healthy population, and < 10 µg/ml (100 nM) in 99% of healthy individuals. Plasma CRP concentrations can, e.g. be measured by homogeneous assay formats or ELISA. C-reactive protein is a marker for underlying systemic inflammation.

Interleukin-6 (IL-6) is a 21 kDa secreted protein that has numerous biological activities that can be divided into those involved in hematopoiesis and into those involved in the activation of the innate immune response. IL-6 is an acute-phase reactant and stimulates the synthesis of a variety of proteins, including adhesion molecules. Its major function is to mediate the acute phase production of hepatic proteins, and its synthesis is induced by the cytokines IL-l and TNF-α. IL-6 is normally produced by macrophages and T lymphocytes. The normal serum concentration of IL-6 is < 5 pg/ml.

Further biomarkers are IL17A, S100A12, osteopontin, pro-MMP-1, pro-MMP-3, hyaluronic acid, sCD14, angiogenesis markers and products of bone, cartilage or synovium metabolism.

The autoantibody which is indicative for spondyloarthritides recognizes the G1 domain of aggrecan (see Zhou et al. (2003), Rheumatology 42:1; doi:10.1093/rheumatology/keg230.

In addition, HLA-B 27, known to be indicative for Morbus Bechterew can be detected, for example, by any nucleic acid amplification technique such as PCR in asample of a subject.

Human Leukocyte Antigen B*27 (subtypes B*2701-2724) is a class I surface antigen encoded by the B locus in the major histocompatibility complex (MHC) on chromosome 6 and presents microbial antigens to T-cells. HLA-B27 is strongly associated with a certain set of autoimmune diseases referred to as the "seronegative spondyloarthropathies".

The autoantibody which is indicative for connective tissue diseases preferably recognizes ds-DNA, SS-A, SS-B, Sm, histone, Scl-70, Jo-1, centromer protein B, RNP, U1-70and/or fodrin.

Antibodies against ds-DNA, SS-A, SS-B, Sm, histone, Scl-70, Jo-1, centromer protein B and RNP are described herein above.

Fodrin (or alpha-fodrin) is also known as spectrin, alpha, non-erythrocytic 1 (alpha-fodrin) or as SPTAN1. It is a major cortical cytoskeletal protein that has been implicated in the establishment of specialized membrane- cytoskeletal domains in differentiating cells.

The autoantibodies which are indicative for vasculitides preferably recognize PR3 and/or MPO.

Proteinase 3 (PR3) is the major autoantigen for anti-neutrophil cytoplasmic antibodies (ANCA) in patients with Wegener's granulomatosis.

Myeloperoxidase (MPO) is an enzyme from the granula of neutrophil granulocytes. A complicated sequence of processing steps (which preclude recombinant production) converts a 80 kDa initial translation product to the mature enzyme which consists of two 64 kDa heavy chains and two 13 kDa light chains. MPO derives its characteristic green color from a unique "green heme" cofactor. The peroxidase activity of MPO generates hypochlorite which acts to kill microbes and to inactivate inhibitors of lytic enzymes which the neutrophil leukocytes releases to degrade material in their vicinity.

MPO is an autoantigen in a number of vasculitides, inflammatory autoimmune diseases of the vasculature, which can be classified depending on the affected type of blood vessel.

Further autoantigens which may be indicative for rheumatic disorders, preferably autoimmune-mediated musculoskeletal disorders can be identified by probing a human protein microarray such as the ProtoArray™. Specifically, sera from subjects diagnosed with an autoimmune-mediated musculoskeletal disorder is profiled on a human protein microarray to identify autoantigens assocaited with autoimmune-mediated musculoskeletal disorders. More specifically, sera from subjects diagnosed with an autoimmune-mediated musculoskeletal disorder are profiled and compared to sera from healthy subjects.

Antigens that were bound by antibodies from sera from subjects diagnosed with an autoimmune-mediated musculoskeletal disorder are candidates for autoantigens associated with autoimmune-mediated musculoskeletal disorder.

In particular, autoantigens which are only identified or more frequently identified in sera from subjects diagnosed with an autoimmune-mediated musculoskeletal disorder and are - in comparison - not or less frequently present in sera from healthy subjects are prime candidate autoantigens which may be applied in the embodiments of the present invention.

Microarrays, or other assay formats, containing these autoantigens are able to detect the presence of autoantibodies in a subject sample that bind these autoantigens, enabling the determination as to whether or not a non-rheumatic disorder can be excluded as cause of a musculoskeletal complaint of a subject or as to whether or not a rheumatic disorder can be excluded as cause of a musculoskeletal complaint of a subject, respectively.

The following Table provides an overview which autoantigens are typically associated with a rheumatic disorder, preferably autoimmune-mediated musculoskeletal disorders. These autoantigens may be applied in the methods, uses and kits of the present invention to determine the presence or absence, respectively, of an autoantibody indicative for an auto-immune mediated musculoskeletal disorder in a sample from a subject.

| Autoimmune-mediated musculoskeletal | Typical marker |
|---|---|
| disorder | |
| Rheumatoid arthritis/ | |
| juvenile rheumatoid arthritis: | anti-CCP, RF, (ANA), CRP, IL-6 |
| Ancylosing spondylitis (M. Bechterew) | HLA-B27, anti-G 1-domain-aggrecan |
| Systemic lupus erythematosus: | ANA, anti-dsDNA, anti-Sm, anti- |
| | RNP/Sm, anti-.SSA, anti-SSB, anti- |
| | histone |
| Systemic sclerosis: | ANA, anti-Scl-70, anti-CENP-B, anti- |
| | RNP/Sm |
| Sjogren syndrome: | ANA, anti-SSA (SSA-52, SSA-60), |
| | anti-SSB, anti-RNP-Sm, anti-fodrin |
| Polymyositis/dermatomyositis : | ANA, anti-Jo-1, anti-RNP/Sm |
| CREST | ANA, anti-CENP-B |
| Mixed connective tissue disease | anti-RNP/Sm, anti-U1-RNP |
| Morbus Wegener | PR3, MPO |
| Infectious arthritis | anti-SLO, anti-HBV, anti-HCV etc. |

Further autoantigens that may be associated with autoimmune-mediated musculoskeletal disorders can be selected from Table 1 of US 2008/0254482. Said Table provides a list of autoantigens that were bound more often by antibodies from sera from RA, SLE and ANCA subjects than by antibodies from healthy individuals. Accordingly, one or more of these autoantigens from said Table is contemplated to be applied in the methods and kits of the present invention.

Accordingly, in a preferred aspect the present invention envisages that at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 40, 45, 50, 55, 60, 65, 70, 75, 80, 85, 90, 95, 100, 120, 140, 150, 160, 180, 200, 220, 240, 250, 260, 280, 300, 400, 500, 600, 700, 800, 900 or 1000 autoantigens are brought into contact with a sample obtained from a subject in order to determine as to whether at least one autoantibody which is indicative for at least one rheumatic disorder, preferably those described herein, is present in said sample, wherein the presence of said autoantibody allows to exclude a non-rheumatic disorder as cause of a musculoskeletal complaint of said subject. The presence is determined by the autoantibody's binding to, reaction with or recognition of an autoantigen in a sample obtained from a subject.

For example, it is envisaged that all autoantigens described herein and/or all known autoantigens associated with a rheumatic disorder and/or those that can and/or will be identified, for example, by applying the methods described in US 2008/0254482 are brought into contact with a sample obtained from a subject in order to determine as to whether at least one autoantibody which is indicative for at least one rheumatic disorder, preferably those described herein, is present in said sample, wherein the presence of said autoantibody allows to exclude a non-rheumatic disorder as cause of a musculoskeletal complaint of said subject. The presence is determined by the autoantibody's binding to, reaction with or recognition of an autoantigen in a sample obtained from a subject can be determined.

Likewise, in another preferred aspect the present invention envisages that at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 40, 45, 50, 55, 60, 65, 70, 75, 80, 85, 90, 95, 100, 120, 140, 150, 160, 180, 200, 220, 240, 250, 260, 280, 300, 400, 500, 600, 700, 800, 900 or 1000 autoantigens or antibodies are brought into contact with a sample obtained from a subject in order to determine as to whether at least one autoantibody or antigen which is indicative for at least one rheumatic disorder, preferably those described herein, is absent in said sample, wherein the absence of said autoantibody or antigen allows to exclude a rheumatic disorder as cause of a musculoskeletal complaint of said subject. The absence is determined by the autoantibody's not binding to, non-reaction with or non-recognition of an autoantigen or antibody of an analyte present in a sample obtained from a subject.

For example, it is envisaged that all autoantigens described herein and/or all known autoantigens associated with a rheumatic disorder and/or those that can and/or will be identified, for example, by applying the methods described in US 2008/0254482 are brought into contact with a sample obtained from a subject in order to determine as to whether at least one autoantibody which is indicative for at least one rheumatic disorder, preferably those described herein, is absent in said sample, wherein the absence of said autoantibody allows to exclude a rheumatic disorder as cause of a musculoskeletal complaint of said subject. The absence is determined by the autoantibody's not binding to, non-reaction with or non-recognition of an autoantigen or antibody of an analyte present in a sample obtained from a subject.

Optionally, the present invention also envisages that proteins and/or polynucleotides contained in a sample from a subject having a musculoskeletal complaint are analyzed for the presence or absence of one or more of the biomarkers mentioned herein and/or commonly known in the art to be associated with a musculoskeletal complaint. For example, it is known that subject having an autoimmune disorder such as Hashimoto thyreoiditis may also have musculoskeletal complaints. Thus, the typical biomarkers for an autoimmune disorder are optionally tested.

"Determining" as used herein in the context of the methods and kits of the present invention refers to identifying the presence or absence or amount of the object to be determined, in particular at least one autoantibody or autoantigen which is indicative for at least one rheumatic disorder. Determining also includes that the sample obtained from a subject is brought into contact (i.e., contacted) with a solid phase which comprises a support and at least one test area, wherein the test area contains an autoantigen or an antibody.

Determining at least one autoantibody or autoantigen which is indicative for at least one rheumatic disorder can be done by any immunoassay known in the art and described hereinabove.

Preferably, determining comprises
i) providing a solid phase which comprises a support and one or more one test areas, the test area each containing an autoantigen,
(ii) contacting a sample obtained from a subject with the solid phase and with at least one autoantibody-specific receptor which carries a signal generating group or is capable of binding to a signal generating group, and
(iii) detecting the presence or/and the amount of the autoantibody by determining the signal generating group on each test area.

Determining in case of determining antigens that may be indicative for a rheumatic disorder may optionally comprise
i) providing a solid phase which comprises a support and one or more one test areas, the test area each containing an antibody (specific for an antigen that may be indicative for a rheumatic disorder),
(ii) contacting a sample obtained from a subject with the solid phase and with at least one antigen-specific receptor (preferably an antibody) which carries a signal generating group or is capable of binding to a signal generating group, and
(iii) detecting the presence or/and the amount of the antigen by determining the signal generating group on each test area.

A preferred solid phase is one having more than one test area. Such a solid phase is also known as array; see, for example, Us 5,432,099 or US 5,126,276. As used herein, the term "array" refers to an arrangement of entities in a pattern on a support. Although the pattern is typically a two-dimensional pattern, the pattern may also be a three-dimensional pattern. In an array of the present invention, the entities are test areas comprising autoantigens.

The term "one or more test areas" includes that the solid phase has at least one test area, for example, has 1, 2, 3, 4, 5, 6, 7, 8, 9, 10, 11, 12, 13, 14, 15, 16, 17, 18 , 19, 20, 21, 22, 23, 24, 25, 26, 27, 28, 29, 30, 48, 50, 52, 54, 56, 58, 60, 70, 72, 74, 76, 78, 80, 82, 84, 86, 88, 90, 92, 94, 96, 98, 100, 102, 104, 106, 108, 110, 120, 128, 130, 140, 150, 160, 170, 180, 190, 200, 250, 256, 384, 512, 1024, 2048, 4096 or more test area(s).

Preferably, a test area contains one autoantigen. However, though less preferred, a test area can contain more than one autoantigen, for example, 2, 3, 4, 5, 6, 7, 8, 9, 10 or more autoantigens.

The autoantigen may be bound to the solid phase by way of a specific binding system such as biotin/avidin, biotin/streptavidin, biotin/antibiotin, hapten/antihapten, carbohydrate/lectin and antibody or antibody fragment and antibody against this antibody or against the antibody fragment. Alternatively, it may be bound by way of covalent interaction, non-covalent interaction, etc.

In certain embodiments, the array can be a microarray or a nanoarray. A "nanoarray" is an array in which separate entities are separated by 0.1 nm to 10 µm, for example from 1 nm to 1 µm.

A "microarray" is an array in the density of entities on the array is at least 100/cm². On microarrays separate entities can be separated, for example, by more than 1 µm.

The "array" as used herein refers to a protein array, a protein microarray or a protein nanoarray to assay for the presence or absence of autoantibodies directed against autoantigens as described herein. For example, it is a custom protein array that includes autoantigens, such as those provided herein, for the determination of the presence o absence of autoantibodies. The term "protein chip" when used herein is used synonymously with protein array or microarray.

Preferably, the array comprises a non-porous carrier. The non-porous carrier may be made of any non-porous material. Preferably, the carrier comprises a surface composed of plastic, glass, meal or metal oxide. Specifically preferred the carrier has a surface composed of polystyrol. It is envisaged that spatially distinct test areas are arranged on the carrier. On these test areas autoantigens are present. Present means that autoantigens are coated onto the test areas or immobilized on the test areas. Immobilization is achieved by, for example, adsorptive binding, covalent coupling or coupling via a highly affine binding of two moieties such as avidin/streptavidin or sugar/lectin.

Alternatively, the array comprises a porous carrier. The carrier may preferably comprise a surface composed of polystyrene such as the microspheres applied in the xMAP technology from Luminex; see WO 97/14028.

It is particularly advantageous if the spatially distinct test areas are loaded with one autoantigen only. Accordingly, for each autoantigen specific conditions can be established such as its concentration, buffer, salt, etc. Thus, it is possible to coat each test area with an autoantigen up to the maximum.

Moreover, no competition of the autoantigens as regards a potential binding to an autoantibody takes place.

The "detecting" step can provide a positive/negative binding result, or can give a value that can be a relative or absolute value for the level of the autoantibody in the sample. The result can provide a diagnosis, prognosis, or be used as an indicator for conducting further tests or evaluation that may or may not result in a diagnosis or prognosis. The detection can be performed on any solid support, such as a bead, dish, plate, well, sheet, membrane, slide, chip, or array, such as a protein array, which can be a microarray, and can optionally be a high density microarray as described herein.

An autoantibody is generally detected by a so-called "indirect test-format", in which the bound autoantibody is detected by an autoantibody-specific receptor.

An "autoantibody-specific receptor" is a moiety that specifically binds to, reacts with or recognizes an autoantibody and carries a signal generating group or is capable of binding to a signal generating group. For example, the moiety can be an antibody against a human autoantibody. The signal generating group can be covalently or non-covalently bound to the autoantibody-specific receptor.

"Non-covalently" includes (a) that the moiety intercalates into the autoantibody; and (b) any kind of suitable binding reaction based on two binding partners which specifically interact with each other in a non-covalent fashion, such as, for example, antigen-antibody binding.

For example, antibodies or antibody fragments can be produced against the autoantibodies and coupled to a signal generating group using conventional antibody technology (see, e.g., Folli et al., 1994, "Antibody-Indocyanin Conjugates for Immunophotodetection of Human Squamous Cell Carcinoma in Nude Mice," Cancer Res. 54:2643-2649; Neri et al., 1997, "Targeting By Affinity-Matured Recombinant Antibody Fragments of an Angiogenesis Associated Fibronectin lsoform," Nature Biotechnology 15:1271-1275). Said antibodies or functional fragments thereof may then bind to the autoantibody.

As the autoantibodies may be present in a sample obtained from a subject, preferably a human subject, the moiety is preferably an antibody specifically binding to the constant region of human antibodies of any lg isotype such lgG, A, M, D or E, with lgG preferred. The constant region contains a constant domain (CH1), a hinge region, and two more constant (CH2 and CH3) domains. Among the lgG isotype, IgGl, G2, G3 and G4 subtypes are known. Accordingly, the antibody is also envisaged to bind to these subtypes. A preferred example of an autoantibody-specific receptor is an antibody or fragment thereof that binds to the Fc portion of an autoantibody. Such an autoantibody-specific receptor could be regarded as so-called universal binder of autoantibodies and could be obtained from a non-human mammal by way of immunization such as a mouse or rat.

An alternative for determination of (auto)antibodies is the so-called double-antigen sandwich (DAGS), in which the autoantigen is used as "solid phase antigen" (bound to a solid phase as described herein) and as "detection antigen" (in liquid phase, i.e., it is additionally added).

In the alternative, the autoantibody-specific receptor is an antigen which is bound by the autoantibody. Preferably, said antigen is the same autoantigen which is bound by the autoantibody and which is present on the test area as described herein elsewhere. More preferably, said antigen is a labelled autoantigen.

If the autoantibody-specific receptor is an autoantigen, the so-called double antigen bridge test/method concept can be applied for determining the presence and/or the amount of the autoantibody contained in the sample from a subject. In such a method the sample is, in addition to the solid phase, incubated with (a) further autoantigen(s) carrying a detectable label. Accordingly, on the solid phase an autoantigen is contained (bound) on a test area and said autoantigen is also present in a form capable of binding to the autoantibody and carries a signal generating group or can bind to a signal generating group.

The autoantibody in the sample liquid is detected by determining the signal generating group bound to the solid phase by means of an immobilized complex.

The test procedure preferably comprises mixing the sample liquid with a purified labelled autoantigen and with the purified autoantigen contained in the test area in order to obtain a labelled immobilized complex consisting of labelled autoantigen, autoantibody and solid phase-bound autoantigen.

The labelled autoantigen that is not contained in the test area carries a signal generating group as described herein. The solid phase autoantigen can be bound for example directly to the solid phase via a bifunctional spacer.

A detailed description of the bridge test format is given in EP-A-0 280 211. This patent application is incorporated by reference.

The detecting step may also be accomplished by a "back titration assay". Specifically, after the (auto)antibody from the sample has bound to its (auto)antigen, a labeled antibody directed against this (auto)antigen is added and will bind to (auto)antigen molecules.which are not covered by the (auto)antibody from the sample and, thus, free autoantibody may be determined in this competitive test procedure, thereby allowing the determination of the amount/concentration of said autoantibody.

The autoantibody-specific receptor carries a "signal generating group". Alternatively, it is capable of binding to a signal generating group. A signal generating group refers to a composition detectable by spectroscopic, photochemical, biochemical, immunochemical, or chemical means. For example, useful labels include radiolabels such as ³²P, ³⁵S, or ¹²⁵l; fluorescent dyes (for example, Cy-3, Cy-5); chromophores, electron-dense reagents; enzymes that generate a detectable signal (e.g., as commonly used in an ELISA); or spin labels. The label or detectable moiety has or generates a measurable signal, such as a radioactive, chromogenic, or fluorescent signal, that can be used to quantify the amount of bound detectable moiety in a sample.

The detectable moiety can be incorporated in or attached to signal generating group either covalently, or through ionic, van der Waals or hydrogen bonds, e.g., incorporation of radioactive nucleotides, or biotinylated nucleotides that are recognized by streptavidin. The label or detectable moiety may be directly or indirectly detectable. Indirect detection can involve the binding of a second directly or indirectly detectable moiety to the detectable moiety. For example, the detectable moiety can be the ligand of a binding partner, such as biotin, which is a binding partner for streptavidin, or a nucleotide sequence, which is the binding partner for a complementary sequence, to which it can specifically hybridize. The binding partner may itself be directly detectable, for example, an antibody may be itself labeled with a fluorescent molecule. The binding partner also may be indirectly detectable.

When the autoantibody-specific receptor is "capable of binding to a signal generating group" this means that it can bind to a signal generating group. For example, the autoantibody-specific receptor may carry a functional group which is able to bind to a signal generating group. A functional group can be streptavidin/avidin which binds to biotin, an antigen such as a tag, for example, GST or histidine residues which binds to an antibody, a sugar which binds a lectin or the known Dig/Anti-Dig system. The moiety that binds to the functional group carries the signal generating group.

An "antigen-specific receptor" applied when determining the presence or absence of an antigen that is indicative for a rheumatic disorder may be an antibody which recognizes the antigen and which carries a signal generating group as described herein.

In a further aspect, the present invention provides a test kit for excluding a non-rheumatic disorder as cause of a musculoskeletal complaint comprising
(a) at least one autoantigen which is indicative for rheumatoid arthritides;
(b) at least one autoantigen which is indicative for connective tissue diseases; and/or
(c) at least one autoantigen which is indicative for vasculitides;
wherein the test kit comprises a non-porous support and at least one test area, the test area each containing an autoantigen which specifically binds the autoantibody to be detected.

Optionally, said kit also comprises at least one antibody for the determination of an antigen which is indicative for spondyloarthritides and/or at least one antigen for the determination of specicifc antibodies which is indicative for infectious arthritides as described herein.

The kits of the present invention may optionally comprise one or more antibodies specific for antigens that may be indicative for a rheumatic disorder.

The kits may optionally also comprise means and methods for detecting autoantigens and/or autoantibodies indicative for autoimmune disorders that may cause musculoskeletal complaints such as Hashimoto disease.

Preferably the kit further comprises at least one control area which does not contain an autoantigen.

The autoantigens of the kit may, for example, be immobilized on a solid support or surface. The autoantigens may, for example, be immobilized in an array. Thus, the kit may be an array including a protein array. The protein microarray may use bead technology, such as the Luminex technology (xMAP) (Luminex Corp., Austin, Tex.). The array can include immobilized on the array one or more positive control proteins, one or more negative controls, and/or one or more normalization controls.

A kit may further comprise a reporter reagent to detect binding of autoantibodies to the autoantigens, such as, for example, an antibody that binds to autoantibody, linked to a detectable label. A kit may further comprise reagents useful for various immune reactivity assays, such as ELISA, or other immunoassay techniques known to those of skill in the art. The assays in which the kit reagents can be used may be competitive assays, sandwich assays, and the label may be selected from the group of well-known labels used for radioimmunoassay, fluorescent or chemiluminescence immunoassay.

The kit can also include a program in computer readable form to analyze results of methods performed using the kits to practice the methods provided herein.

The kits of the present invention may also comprise one or more of the components in any number of separate containers, packets, tubes, vials, microtiter plates and the like, or the components may be combined in various combinations in such containers.

The kits of the present invention may also comprise instructions for performing one or more methods described herein and/or a description of one or more compositions or reagents described herein. Instructions and/or descriptions may be in printed form and may be included in a kit insert. A kit also may include a written description of an Internet location that provides such instructions or descriptions.

In another aspect, the present invention provides for the use of the (test)kit of the present invention for excluding a non-rheumatic disorder as cause of a musculoskeletal complaint of a subject. The use is preferably for diagnosis.

Also, the present invention provides for a method of diagnosis for excluding a non-rheumatic disorder as cause of a musculoskeletal complaint of a subject.

The embodiments described hereinabove, for example, with the methods of the present invention are equally applicable to the kits and uses, mutatis mutandis.

The disclosure of the present invention may best be understood in conjunction with the accompanying drawings, incorporated herein by references. Furthermore, a better understanding of the present invention and of its many advantages will be had from the following examples, given by way of illustration and are not intended as limiting.

### Figure

The figures show:
Figure 1: Algorithm for the diagnosis of musculoskeletal complaints (taken from Harrison's Principles of Internal MedicineHarrison's Chapter 320).
   ESR: erythrocyte sedimentation rate; CRP: C-reactive protein; DIP: distal interphalangeal; CMC: carpometacarpal; PIP: proximal interphalangeal;
   MCP: metacarpophalangeal; MTP: metatarsophalangeal; PMR: polymyalgia rheumatica; SLE: systemic lupus erythematosus; JA: juvenile arthritis

### Examples

The following examples illustrate the invention. These examples should not be construed as to limit the scope of this invention. The examples are included for purposes of illustration and the present invention is limited only by the claims.

### Example 1: Immunological multiparametric chip technique IMPACT - general procedure

A black Polystyrene chip with a surface area of about 2.5 x 6 mm is coated completely with a streptavidin layer. Onto this streptavidin surface we apply lines of identical reagent spots (about 20 spots per line and reagent) using ink-jet technology. Each spot is about 150 µm in diameter and contains biotinylated binding reagents capable of specifically binding with an analyte (e.g., antigens or antibodies) in a sample.

Each sample is diluted with a sample dilution buffer and 40 µl of diluted sample is applied per chip and incubated. The assay is performed on an automated pre- prototype instrument.

### Assay specific reagents

| | |
|---|---|
| Sample dilution buffer: | 50mM Tris, 30mM MES, 50mM NaCl, 5mM EDTA, 0.5% |
| | Casein, 0.1 % detergent (Polydocanol), 0,2% preservative |
| | (Oxypyrion and Methylisothiazol-Hydrocloride (MIT)) |
| Washing buffer: | 10mM Tris, 0.01 % Polydocanol, 0.001 % Oxypyrion, |
| | 0.001% MIT |

### Assay procedure

The diluted sample is incubated for 6 min at 37°C. During this incubation analytes contained in the sample binds to its specific binding reagent. The sample is then aspirated and the chip is washed using washing buffer. Afterwards the chip is incubated for 3 min at 37°C with a mixture of conjugate antibodies which are digoxigenylated and which specifically bind to the analytes from the sample that are bound to the binding partners located in the spots. After a further washing step the chip is incubated with a fluorescence labeled <Dig> antibody for 3min. at 37°C. After a further washing step and aspiration of surplus reagent the fluorescence label is excited, light is detected by a CCD-camera and light intensity is transformed into analyte concentrations.

### Example 2: Composition of the multiparametric arrays

Following multiplex array kits were produced and tested with the sample panel to determine following analytes:
Multiplex-1: RF-IgM, CRP, SAA
Multiplex-2: 5 different anti-CCP-autoantibodies: 5 different peptides (cyclic citrullinated peptides) are coated onto the surface
Multiplex-3: anti-Centromer B peptide, anti-SSA52, anti-Jo-1, anti-SSA60, anti-SSB, anti-Scl70, anti-RNP/Sm, anti-dsDNA
Multiplex 4: lL-6, Hyaluronic acid (HA) pro-MMP-3, E-Selectin

### Example 3: Study population

### Patients

Patients were enrolled in six European centres in a prospective longitudinal study following unified standard operational procedures (SOP). While samples from patients with osteoarthritis (OA) were collected in Bristol (UK), samples from patients with autoimmune diseases and controls were collected in Chur (CH), the sample from patients with RA were collected in five centres. In this study only the samples at the time of enrolment were used. Inclusion criteria for the RA cohort were the presence of a minimum of four ACR criteria plus a retrospective confirmation of the diagnosis after 2 years follow-up. (Hochberg, M.C., et al., Arthritis Rheum. 35 (1992) 498-502). All RA, autoimmune and control patients had established disease with a positive diagnosis confirmed by the respective centre. Ethics approval for this study was given by the ethic committees of each of the participating centres.

### Case report forms

Standardized case report forms (CRF) of RA patients registered age, sex, ACR criteria, disease duration, x-rays of hands and feet, joint surgeries and comorbidities, tender and swollen joint count, past and present medication, the Health Assessment Questionnaire (HAQ) and the SF36 Questionnaire.

Standardized case report forms for autoimmune and control diseases reported age, sex, start of first symptoms, final diagnosis, clinical signs, CRP, medication and joint association.

### Panel composition for diagnostic setting

The composition of the sample collective was modelled to mirror the diagnostic setting at a general practitioner. The representation of the different disease groups likely to be encountered by a general practitioner is based on the relative prevalence data of musculoskeletal diseases in the general population of the USA (Lawrence et. al. 1998) and is only modestly preselected. Positive results of the assay will lead to a referral of the respective patients to a specialized clinic.

**Table 1: Composition of the sample panel (n = 848) and representation of the disease groups in the GP setting according to the prevalences**

| **Collective** | **Patient sub group** | **No samples** | **Prevalence (%)** | **Sample size in simulation (in one loop)** |
|---|---|---|---|---|
| Diseased | RA | 367 | 32.8 | 328 |
| | Connective tissue diseases | 23 | 4.2 | 43 |
| | Systemic Sclerosis/Sjögren | 24 | 13.0 | 131 |
| | | | | |
| Controls | Crystal induced synovitis | 24 | 1.5 | 15 |
| | OA | 286 | 22.2 | 222 |
| | Low back pain | 104 | 23.6 | 236 |
| | Fibromyalgia | 20 | 2.7 | 27 |
| | | | | |

### Example 4: Univariate results of single markers

The accuracy of each diagnostic single marker is described by its receiver-operating characteristics (ROC) (see especially Zweig, M.H., and Campbell, G., Clin. Chem. 39 (1993) 561-577). The ROC graph is a plot of all of the sensitivity/specificity pairs resulting from continuously varying the decision thresh-hold over the entire range of data observed. The ROC-area and the sensitivity at a specificity of 90% were determined for each single marker.

**Table 2: Univariate results (Median of 100 simulation loops) on the modelling panel diagnosed with "Disease" (autoimmune incl. RA) versus "Controls"**

| **Single marker** | **AUC (%)** | **Sensitivity (%)** |
|---|---|---|
| Anti-CCP-29 | 75 | 56 |
| Anti-CCP-31 | 74 | 57 |
| Anti-CCP-37 | 74 | 55 |
| Anti-CCP-3A | 76 | 57 |
| Anti-CCP-MP1 | 76 | 57 |
| **Anti-CCP-max** | **74** | **55** |
| Scl 70 | 53 | 17 |
| SSA 60 | 57 | 23 |
| SSA 52 | 59 | 28 |
| SSB | 62 | 26 |
| Jo-1 | 54 | 14 |
| Centromer B | 59 | 21 |
| ds DNA | 63 | 19 |
| RNP/Sm | 63 | 22 |
| **ANA-max** | **65** | **35** |
| CRP | 65 | 29 |
| HA | 50 | 18 |
| IL-6 | 70 | 43 |
| SAA | 63 | 34 |
| RF-Ig M | 76 | 56 |
| Pro-MMP-3 | 59 | 34 |
| E-Selectin | 53 | 16 |

Due to the higher prevalence of RA patients within the "diseased group" the CCP peptides and the rheumatoid factor show the highest sensitivities. Since the goal of such a referral test is to detect all autoimmune diseases without any differential diagnosis, we evaluated also an anti-CCP-max or an ANA-max score. This max-score is defined as the concentration of the antigen with the highest value within the group of similar antigens like anti-ccp-peptides or all ANA auto-antigens.

### Example 5: Multivariate Analysis

The patient cohorts were randomly split into a training set and a test set (app. 50 % of the patients each). On the training set a classification algorithm was develop and on the independent test set the algorithm was validated. The sample numbers of training and test set can be seen in Table 3.

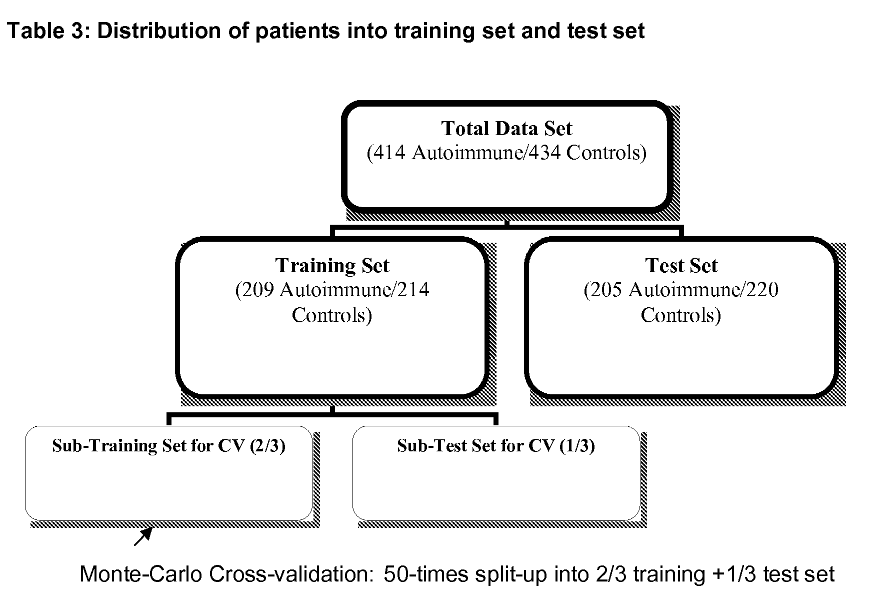

The classification algorithms were generated with the Regularized Discriminant Analysis (RDA), which is a generalization of the common Discriminant Analysis, i.e. Quadratic-and Linear Discriminant Analysis (McLachlan, G.J., Discriminant Analysis and Statistical Pattern Recognition, Wiley Series in probability and mathematical statistics, 1992). In the RDA alternatives to the usual maximum likelihood (plug-in) estimates for the covariance matrices are used. These alternatives are characterized by two parameters (*λ,γ*), the values of which are customized to individual situations by jointly minimizing a sample-based estimate of future misclassification risk (Friedman, J.H., Regularized Discriminant Analysis, Journal of the American Statistical Association, Vol. 84, 1989, 165-175). The marker panels were stepwise constructed starting from the best single marker for the classification problem and ending when the total classification error do not change remarkable any more. In order to gain centralized distributions every single marker was transformed with the natural logarithmic function. 50-fold cross validation was used on the training set to get robust estimates of the total error (sensitivity, specificity). Once the marker panel was defined, it was validated without any further adjustment with an independent test set.

Table 4 represents the classification results of the modelling panel of diseased patients (autoimmune diseases including RA) versus the control collective. The best 2 marker panel selected in the training set was anti-CCP-max and ANA-max. The addition of a third marker (IL-6 or SAA) shows comparable results to the best 2-marker panel: a slight increase of the sensitivity accompanied by a slight decrease of the specificity. After addition of a further marker the number of false positive or false negative classified patients was increased.

The best marker panels were then proven by transfer of the algorithm to the independent test set of patients. As shown in Table 4 the results of the 2 marker panel can be reproduced in the test set. This supports that the algorithm developed by combination of anti-ccp max and ANA max is very robust. On the other hand the algorithm developed with 3 markers in the training set cannot be transferred to the test set without loss of sensitivity (marker panel incl. IL-6) or specificity (marker panel incl. SAA).

The aim of the current invention was to improve the sensitivity for the detection of all autoimmune diseases without any differential diagnosis within a collective of musculoskeletal disorders. The best marker panel with the lowest total error in training and test set was the panel of anti-CCP-max and ANA-max. Therefore it is obvious for the best detection of an autoimmune disease to include as much as possible specific antigens for the detection of autoimmune diseases or of RA and to use the maximum signal or concentration for integration in an algorithm. In our specific case 5 different CCP-peptides and 8 different ANAs were used to achieve the best results.

**Table 4: Classification results on the modelling panel diagnosed with "Disease" (RA + autoimmune) versus "Controls"**

| **No of Markers** | **marker or marker panel** | **Training Set Cross validation (50 fold)** | | | **Test Set** | |
|---|---|---|---|---|---|---|
| | | **Total Error** | **Sensitivity (%)** | **Specificity (%)** | **Sensitivity (%)** | **Specificity (%)** |
| 1 | log RF-IgM | 0.29 | 53 | 91 | 52 | 96 |
| 1 | log anti-CCP-max | 0.27 | 52 | 95 | 53 | 88 |
| **2** | **log anti-CCP-max log ANA-max** | **0.18** | **72** | **93** | **75** | **87** |
| 3 | log anti-CCP-max log ANA-max log IL-6 | 0.18 | 73 | 91 | 61 | 89 |
| 3 | log anti-CCP-max log ANA-max log SAA | 0.18 | 73 | 91 | 80 | 84 |
| 4 | log anti-CCP-max log ANA-max log IL-6 log pro-MMP3 | 0.19 | 71 | 91 | - | - |

The present invention may also be characterized by the following items:
1. A method for excluding a non-rheumatic disorder as cause of a musculoskeletal complaint of a subject comprising determining as to whether at least one autoantibody or antigen which is indicative for at least one rheumatic disorder is present in a sample obtained from the subject,
   wherein the presence of said autoantibody or antigen allows to exclude a non-rheumatic disorder as cause of a musculoskeletal complaint of a subject.
2. The method of the preceding item, wherein the determining comprises
   i) providing a solid phase which comprises a support and one or more test areas, the test area each containing an autoantigen,
   (ii) contacting a sample obtained from a subject with the solid phase and with at least one autoantibody-specific receptor which carries a signal generating group or is capable of binding to a signal generating group, and
   (iii) detecting the presence or/and the amount of the autoantibody by determining the signal generating group on each test area.
3. The method of any of the preceding items, whereby a signal is classified as positive when it is above a predetermined test-area-specific cut-off value and is classified as negative when it is below a predetermined test-area-specific cut-off value,
   wherein a positive signal excludes a non-rheumatic disorder as cause of a musculoskeletal complaint.
4. The method of the preceding item, wherein said cut-off value is determined individually for each test area.
5. The method of any of the preceding items, wherein said non-rheumatic disorder is selected from the group consisting of osteoarthritis, osteoporosis, osteonecrosis, osteopenia, fibromyalgia, low back pain, (articular) gout, pseudogout, bursitis, trauma, fracture, tendinitis.
6. The method of any of the preceding items, wherein said rheumatic disorder includes rheumatoid arthritides, connective tissue diseases and/or vasculitides.
7. The method of any of the preceding items, wherein said autoantibody is indicative for rheumatoid arthritides, connective tissue diseases and/or vasculitides.
8. The method of any of the preceding items, wherein said rheumatoid arthritides include rheumatoid arthritis and/or juvenile rheumatoid arthritis.
9. The method of any of the preceding items, wherein said autoantibody indicative for rheumatoid arthritides recognizes citrullinated peptides, ANA and/or RF.
10. The method of item 9 further comprising determining the amount of IL-6 and/or CRP.
11. The method of any of the preceding items, wherein said connective tissue diseases include systemic lupus erythematosus, systemic sclerosis, polymyositis, dermatomyositis, Sjogren syndrome, CREST-syndrome and/or mixed connective tissue disease.
12. The method of any of the preceding items, wherein said autoantibody indicative for connective tissue diseases recognizes ds-DNA, SS-A, SS-B, Sm, histone, Scl-70, Jo-1, centromer protein B, RNP and/or fodrin.
13. The method of any of the preceding items, wherein said vasculitides include polymyalgia rheumatica, giant cell arteritis, Morbus Wegener and/or Behcet's syndrome.
14. The method of any of the preceding items, wherein said autoantibody indicative for vasculitides recognizes PR3 and/or MPO.
15. The method of any of the preceding items further comprising determining as to whether at least one antigen or (auto)antibody which is indicative for at least one spondyloarthritides disorder and/or infectious arthritides disorder is present in a sample obtained from the subject.
16. A test kit for excluding a non-rheumatic disorder as cause of a musculoskeletal complaint comprising
   (a) at least one autoantigen which is indicative for rheumatoid arthritides;
   (b) at least one autoantigen which is indicative for connective tissue diseases; and/or
   (c) at least one autoantigen which is indicative for vasculitides;
   wherein the test kit comprises a non-porous support and at least one test area, the test area each containing an autoantigen which specifically binds the autoantibody to be detected.
17. The test kit of the preceding item, which further comprises at least one control area which does not contain an autoantigen.
18. Use of the test kit of any of the preceding items for excluding a non-rheumatic disorder as cause of a musculoskeletal complaint of a subject.

Having now fully described the present invention in some detail by way of illustration and examples for purposes of clarity of understanding, it will be obvious to one of ordinary skill in the art that the same can be performed by modifying or changing the invention within a wide and equivalent range of conditions, formulations and other parameters without affecting the scope of the invention or any specific embodiment thereof, and that such modifications or changes are intended to be encompassed within the scope of the appended claims.

One of ordinary skill in the art will appreciate that starting materials, reagents, purification methods, materials, substrates, device elements, analytical methods, assay methods, mixtures and combinations of components other than those specifically exemplified can be employed in the practice of the invention without resort to undue experimentation. All art-known functional equivalents, of any such materials and methods are intended to be included in this invention. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

As used herein, "comprising" is synonymous with "including," "containing," or "characterized by," and is inclusive or open-ended and does not exclude additional, unrecited elements or method steps. As used herein, "consisting of" excludes any element, step, or ingredient not specified in the claim element. As used herein, "consisting essentially of" does not exclude materials or steps that do not materially affect the basic and novel characteristics of the claim. In each instance herein any of the terms "comprising", "consisting essentially of" and "consisting of" may be replaced with either of the other two terms.

When a group of materials, compositions, components or compounds is disclosed herein, it is understood that all individual members of those groups and all subgroups thereof are disclosed separately. When a Markush group or other grouping is used herein, all individual members of the group and all combinations and subcombinations possible of the group are intended to be individually included in the disclosure. Every formulation or combination of components described or exemplified herein can be used to practice the invention, unless otherwise stated. Whenever a range is given in the specification, for example, a temperature range, a time range, or a composition range, all intermediate ranges and subranges, as well as all individual values included in the ranges given are intended to be included in the disclosure. In the disclosure and the claims, "and/or" means additionally or alternatively. Moreover, any use of a term in the singular also encompasses plural forms.

All references cited herein are hereby incorporated by reference in their entirety to the extent that there is no inconsistency with the disclosure of this specification. Some references provided herein are incorporated by reference to provide details concerning sources of starting materials, additional starting materials, additional reagents, additional methods of synthesis, additional methods of analysis, additional biological materials, additional nucleic acids, chemically modified nucleic acids, additional cells, and additional uses of the invention. All headings used herein are for convenience only. All patents and publications mentioned in the specification are indicative of the levels of skill of those skilled in the art to which the invention pertains, and are herein incorporated by reference to the same extent as if each individual publication, patent or patent application was specifically and individually indicated to be incorporated by reference. References cited herein are incorporated by reference herein in their entirety to indicate the state of the art as of their publication or filing date and it is intended that this information can be employed herein, if needed, to exclude specific embodiments that are in the prior art. For example, when composition of matter are claimed, it should be understood that compounds known and available in the art prior to Applicant's invention, including compounds for which an enabling disclosure is provided in the references cited herein, are not intended to be included in the composition of matter claims herein.

## Claims

1. A method for excluding a non-rheumatic disorder as cause of a musculoskeletal complaint of a subject comprising determining as to whether at least one autoantibody or antigen which is indicative for at least one rheumatic disorder is present in a sample obtained from the subject,
wherein the presence of said autoantibody or antigen allows to exclude a non-rheumatic disorder as cause of a musculoskeletal complaint of a subject.

2. The method of the preceding claim, wherein the determining comprises
i) providing a solid phase which comprises a support and one or more test areas, the test area each containing an autoantigen,
(ii) contacting a sample obtained from a subject with the solid phase and with at least one autoantibody-specific receptor which carries a signal generating group or is capable of binding to a signal generating group, and
(iii) detecting the presence or/and the amount of the autoantibody by determining the signal generating group on each test area.

3. The method of any of the preceding claims, whereby a signal is classified as positive when it is above a predetermined test-area-specific cut-off value and is classified as negative when it is below a predetermined test-area-specific cut-off value,
wherein a positive signal excludes a non-rheumatic disorder as cause of a musculoskeletal complaint.

4. The method of any of the preceding claims, wherein said non-rheumatic disorder is selected from the group consisting of osteoarthritis, osteoporosis, osteonecrosis, osteopenia, fibromyalgia, low back pain, (articular) gout, pseudogout, bursitis, trauma, fracture, tendinitis.

5. The method of any of the preceding claims, wherein said rheumatic disorder includes rheumatoid arthritides, connective tissue diseases and/or vasculitides.

6. The method of any of the preceding claims, wherein said autoantibody is indicative for rheumatoid arthritides, connective tissue diseases and/or vasculitides.

7. The method of any of the preceding claims, wherein said rheumatoid arthritides include rheumatoid arthritis and/or juvenile rheumatoid arthritis.

8. The method of any of the preceding claims, wherein said autoantibody indicative for rheumatoid arthritides recognizes citrullinated peptides, ANA and/or RF.

9. The method of any of the preceding claims, wherein said connective tissue diseases include systemic lupus erythematosus, systemic sclerosis, polymyositis, dermatomyositis, Sjogren syndrome, CREST-syndrome and/or mixed connective tissue disease.

10. The method of any of the preceding claims, wherein said autoantibody indicative for connective tissue diseases recognizes ds-DNA, SS-A, SS-B, Sm, histone, Scl-70, Jo-1, centromer protein B, RNP and/or fodrin.

11. The method of any of the preceding claims, wherein said vasculitides include polymyalgia rheumatica, giant cell arteritis, Morbus Wegener and/or Behcet's syndrome.

12. The method of any of the preceding claims, wherein said autoantibody indicative for vasculitides recognizes PR3 and/or MPO.

13. The method of any of the preceding claims further comprising determining as to whether at least one antigen or (auto)antibody which is indicative for at least one spondyloarthritides disorder and/or infectious arthritides disorder is present in a sample obtained from the subject.

14. A test kit for excluding a non-rheumatic disorder as cause of a musculoskeletal complaint comprising
(a) at least one autoantigen which is indicative for rheumatoid arthritides;
(b) at least one autoantigen which is indicative for connective tissue diseases; and/or
(c) at least one autoantigen which is indicative for vasculitides;
wherein the test kit comprises a non-porous support and at least one test area, the test area each containing an autoantigen which specifically binds the autoantibody to be detected.

15. Use of the test kit of any of the preceding claims for excluding a non-rheumatic disorder as cause of a musculoskeletal complaint of a subject.
